(19) Europäisches Patentamt
European Patent Office
Office européen des brevets

(11) Veröffentlichungsnummer: **0 283 876 B1**

# (12) EUROPÄISCHE PATENTSCHRIFT

(45) Veröffentlichungstag der Patentschrift: **20.07.94**

(51) Int. Cl.$^5$: **C07D 249/12**, C07D 249/14, C07D 405/12, C07D 401/12, C07D 409/12, C07D 413/12, C07D 417/12, C07D 403/06, A01N 43/653, A01N 43/74, A01N 43/80

(21) Anmeldenummer: **88103871.5**

(22) Anmeldetag: **11.03.88**

Teilanmeldung 93117477.5 eingereicht am 11/03/88.

Die Akte enthält technische Angaben, die nach dem Eingang der Anmeldung eingereicht wurden und die nicht in dieser Patentschrift enthalten sind.

## (54) Substituierte Triazolinone.

(30) Priorität: **24.03.87 DE 3709574**

(43) Veröffentlichungstag der Anmeldung: **28.09.88 Patentblatt 88/39**

(45) Bekanntmachung des Hinweises auf die Patenterteilung: **20.07.94 Patentblatt 94/29**

(84) Benannte Vertragsstaaten: **BE CH DE ES FR GB IT LI NL**

(56) Entgegenhaltungen:
**EP-A- 0 140 194**
**EP-A- 0 213 718**
**EP-A- 0 294 666**
**EP-A- 0 298 371**
**EP-A- 0 305 844**
**DE-A- 2 042 660**
**DE-A- 2 042 660**
**DE-A- 2 707 801**
**DE-A- 2 707 801**
**GB-A- 919 458**
**US-A- 3 308 131**
**US-A- 3 952 001**

**TETRAHEDRON, Band 33, 1977, Seiten 1999-2006**

(73) Patentinhaber: **BAYER AG**
**D-51368 Leverkusen(DE)**

(72) Erfinder: **Findeisen, Kurt, Dr.**
**In der Follmühle 10**
**D-5068 Odenthal 2(DE)**
Erfinder: **Lindig, Markus, Dr.**
**Dahlienweg 16**
**D-4010 Hilden(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**
Erfinder: **Strang, Harry, Dr.**
**Unterdorfstrasse 6A**
**D-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 283 876 B1

PATENT ABSTRACTS OF JAPAN, Band 3, Nr. 1, 24. Januar 1979, Seite 134 C 35

PATENT ABSTRACTS OF JAPAN, Band 2, Nr. 24, 16. Februar 1978, Seite 4198 C 77

PATENT ABSTRACTS OF JAPAN, Band 8, Nr. 130, 16. Juni 1984, Seite C-229 1567

## Beschreibung

Die Erfindung betrifft neue substituierte Triazolinone, mehrere Verfahren zu ihrer Herstellung und ihre Verwendung als Herbizide und Pflanzenwachstumsregulatoren.

Es ist bekannt, daß bestimmte Stickstoffheterocyclen wie beispielsweise das Imidazolidin-2-on-1-carbonsäureisobutylamid (vgl. z.B. K. H. Büchel "Pflanzenschutz und Schädlingsbekämpfung" S. 170, Thieme Verlag Stuttgart 1977) oder das 1-Phenyl-3-(3-trifluormethylphenyl)-5-methyl-perhydropyrimidin-2-on (vgl. z. B. EP-A-58 868 oder DE-A-3 237 479) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinone, wie beispielsweise das 1-(N,N-Dimethylcarbamoyl)-3-isopropylthio-4-methyl-1,2,4-triazolin-5-thion oder das 1-(N,N-Dimethylcarbamoyl)-3-ethylthio-4-methyl-1,2,4-triazolin-5-thion oder das 1-(N,N-Dimethylcarbamoyl)-3-isopropylthio-4-methyl-1,2,4-triazolin-5-on oder das 1-(N,N-Dimethylcarbamoyl)-3-ethylthio-4-methyl-1,2,4-triazolin-5-on oder das 1-(N,N-Dimethylcarbamoyl)-3-methylthio-4-methyl-1,2,4-triazolin-5-on oder das 1-(N,N-Dimethylcarbamoyl)-3-propylthio-4-methyl-1,2,4-triazolin-5-thion oder das 1-(N,N-Dimethylcarbamoyl)-3-allylthio-4-methyl-1,2,4-triazolin-5-thion oder das 1-(N,N-Dimethylcarbamoyl)-3-methylthio-4-methyl-1,2,4-triazolin-5-thion bekannt (vgl. DE-A-2 707 801). Über eine Wirksamkeit dieser vorbekannten Triazolinone als Herbizide oder Pflanzenwachstumsregulatoren ist bisher nichts bekannt.

Weitere Triazolinone werden in Tetrahedron 22, 1999-2006 (1977) beschrieben und hier insbesondere die Verbindungen
2(H)-Carbamoyl-3-hydroxy-5-methylthio-1,2,4-triazol, 5-Benzylthio-2(H)-carbamoyl-3-hydroxy-1,2,4-triazol, und 5-Benzylthio-3-hydroxy-2(H)-phenylcarbamoyl-1,2,4-triazol.

Außerdem werden Triazolinone, jedoch meist mit anderem Substitutionsmuster am Triazolring in folgenden Druckschriften offenbart:

| | |
|---|---|
| DE-A-2 042 660 | unter Hinweis auf pflanzenschützende Eigenschaften |
| US-A-3 308 131 | unter Hinweis auf breite Verwendungsmöglichkeiten wie Herbizide, Gummiadditive etc, |
| JP-A-53 135 981 und JP-A-52 125 168 | beide mit Hinweisen auf herbizide Verwendbarkeit. |

Schließlich seien noch die Patentschriften EP-A-294 666, EP-A-298 371 und EP-A-305 844 genannt, die zum Zeitpunkt der Anmeldung der vorliegenden Patentanmeldung nicht veröffentlicht waren, jedoch alle 1,2,4-trisubstituierte Triazolinone mit herbiziden Eigenschaften offenbaren.

Es wurden neue substituierte Triazolinone der allgemeinen Formel (I),

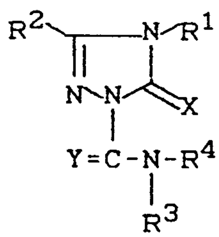

(I)

in welcher

$R^1$ für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,

$R^2$ für einen Rest

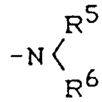

oder für einen Rest $S(O)_n$-$R^7$ steht,

$R^3$ und $R^4$ unabhängig voneinander jeweils für Wasserstoff, Methyl, Ethyl, n- oder i-Propyl, n-, i-, s- oder t-Butyl, n- oder i-Pentyl, n - oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 2 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden durch Fluor,Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen; außerdem für im Heterocyclylteil gegebenenfalls ein- bis dreifach, gleich oder verschieden durch Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl mit den Heterocyclen

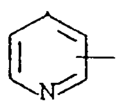 ; 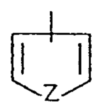 ; 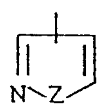 ; 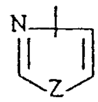 ;

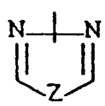 ; 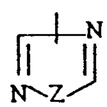 ; 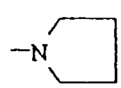 ; 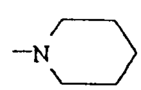 ;

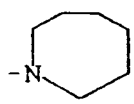 ; 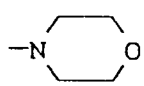 oder 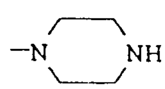

stehen,

wobei Z jeweils für Sauerstoff oder Schwefel steht,

außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl,

Phenylcyanpropyl, Benzyloxy, Phenylethyloxy, Phenoxy, Benzoyl, Phenyl oder Naphthyl stehen, oder

$R^3$ und $R^4$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

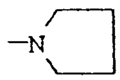 ; 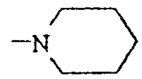 ; 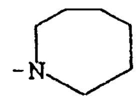 ; 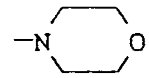 ;

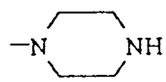 ; 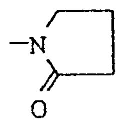 ; 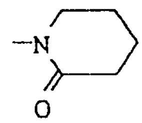 ; 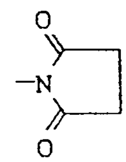 ;

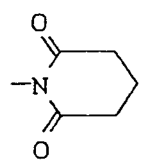 ; 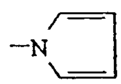 oder 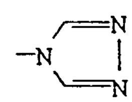

stehen,

X für Sauerstoff oder Schwefel steht und

Y für Sauerstoff oder Schwefel steht, wobei

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder

$R^5$ und $R^6$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

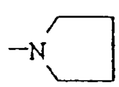 ; 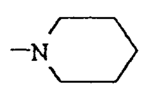 ; 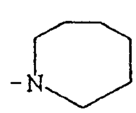 ; 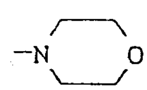 ;

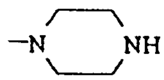 ; 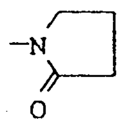 ; 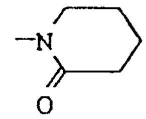 ; 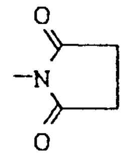 ;

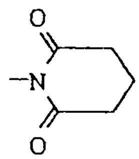 ; 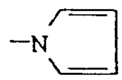 oder 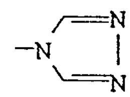

stehen,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und

n für eine Zahl 0, 1 oder 2 steht,

wobei jedoch $R^2$ nur dann für einen Rest $-S(O)_n-R^7$ steht, wenn $R^3$ und $R^4$ nicht gleichzeitig für Methyl

stehen, ausgenommen die Verbindungen 5-Benzylthio-3-hydroxy-2(H)-phenylcarbamoyl-1,2,4-triazol, 2(H)-Carbamoyl-3-hydroxy-5-methylthio-1,2,4-triazol und 5-Benzylthio-2(H)-carbamoyl-3-hydroxy-1,2,4-triazol.

Im einzelnen sei auf die bei den Herstellungsbeispielen genannten Verbindungen verwiesen.

Verwendet man beispielsweise 1-Chlorcarbonyl-3-dimethylamino-4-methyl-1,2,4-triazolin-5-on und Allylamin als Ausgangsstoffe, so läßt sich der Reaktionsablauf des erfindungsgemäßen Verfahrens (a) durch das folgende Formelschema darstellen:

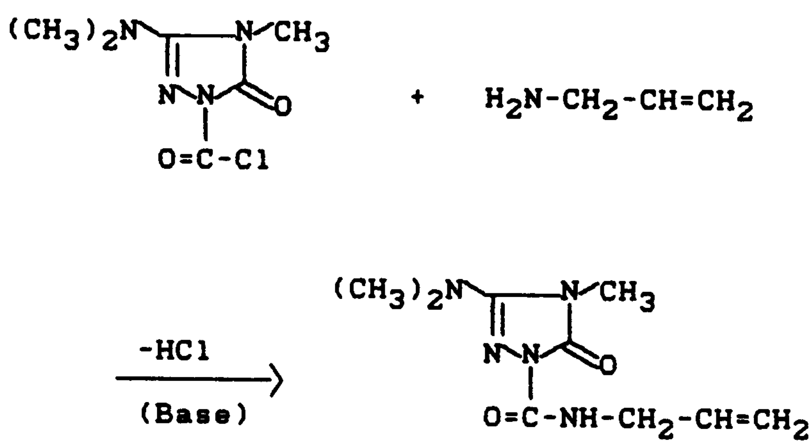


Verwendet man beispielsweise 3-Dimethylamino-4-methyl-1H-1,2,4-triazolin-5-on und Isopropylisocyanat als Ausgangsstoffe, so läßt sich der Reaktionsablauf des erfindungsgemäßen Verfahrens (b) durch das folgende Formelschema darstellen:

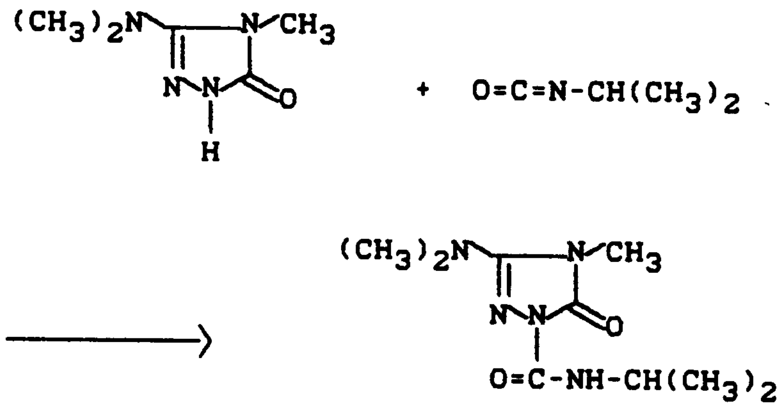

Die zur Durchführung des erfindungsgemäßen Verfahrens (a) als Ausgangsstoffe benötigten Chlor(thio)-carbonyltriazolinone sind durch die Formel (II) allgemein definiert. In dieser Formel (II) stehen $R^1$, $R^2$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Chlor(thio)carbonyltriazolinone der Formel (II) sind noch nicht bekannt.
Man erhält sie, wenn man in 1-Stellung unsubstituierte Triazolinone der Formel (IV),

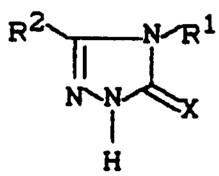

(IV)

in welcher
$R^1$, $R^2$ und X die oben angegebene Bedeutung haben,
mit (Thio)Phosgen der Formel (VI),

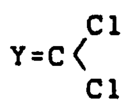

(VI)

in welcher
Y die oben angegebene Bedeutung hat,
gegebenenfalls in Gegenwart eines Verdünnungsmittels wie beispielsweise Toluol oder Acetonitril und gegebenenfalls in Gegenwart eines Säurebindemittels wie beispielsweise Triethylamin bei Temperaturen zwischen + 20 °C und + 150 °C umsetzt,
Chlor(thio)carbonylverbindungen der Formel (IIa),

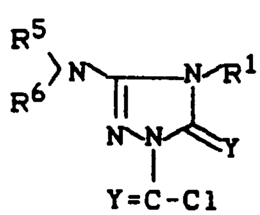

(IIa)

in welcher
$R^1$, $R^5$, $R^6$ und Y die oben angegebene Bedeutung haben,

erhält man alternativ auch, wenn man Aminoguanidiniumhydrochloride der Formel (VII),

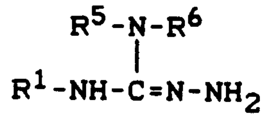 x HCl (VII)

in welcher

$R^1$, $R^5$ und $R^6$ die oben angegebene Bedeutung haben,

mit doppelt-molarem Überschuß an (Thio)Phosgen der Formel (VI),

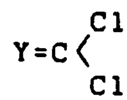 (VI)

in welcher

Y die oben angegebene Bedeutung hat,

gegebenenfalls in Gegenwart eines Verdünnungsmittels wie beispielsweise Toluol oder Acetonitril und gegebenenfalls in Gegenwart eines Säurebindemittels bei Temperaturen zwischen + 20 °C und + 150 °C umsetzt.

Die Aminoguanidiniumhydrochloride der Formel (VII) erhält man in Analogie zu bekannten Verfahren, beispielsweise wenn man die allgemein bekannten Harnstoffe der Formel (VIII),

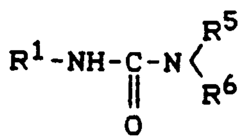 (VIII)

in welcher

$R^1$, $R^5$ und $R^6$ die oben angegebene Bedeutung haben,

zunächst in einer 1. Stufe mit (Thio)Phosgen der Formel (VI),

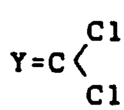 (VI)

in welcher

Y die oben angegebene Bedeutung hat,

gegebenenfalls in Gegenwart eines Verdünnungsmittels wie beispielsweise Toluol oder Acetonitril bei Temperaturen zwischen + 10 °C und + 150 °C umsetzt, und die so erhältlichen Formamidinhydrochloride der Formel (IX),

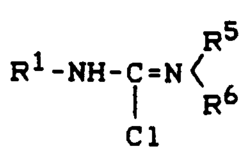 x HCl (IX)

in welcher

$R^1$, $R^5$ und $R^6$ die oben angegebene Bedeutung haben,

in einer 2. Stufe mit Hydrazinhydrat, gegebenenfalls in Gegenwart eines Verdünnungsmittels wie beispielsweise Isopropanol oder Dichlormethan bei Temperaturen zwischen - 10 °C und + 60 °C umsetzt (vgl. z.B. J. org. Chem. 19, 1807 [1954]; Bull. Soc. Chim. Fr. 1975, 1649; US 2 845 458).

Harnstoffe der Formel (VIII), Phosgen und Thiophosgen der Formel (VI) sind allgemein bekannte Verbindungen der organischen Chemie.

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten in 1-Stellung unsubstituierten Triazolinone sind durch die Formel (IV) allgemein definiert. In dieser Formel (IV) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolinone der Formel (IV) sind teilweise bekannt (vgl. z.B. Chem. Ber. 102, 735 [1969]; Chem. Ber. 107, 454 [1974]; Arch. Pharm. 307, 509 [1974]; Helv. Chim. Acta 63, 841 [1980]; US-A-4 098 896; US-A-4 110 332; US-A-4 530 898; DE-A-2 250 572; J. chem. Soc. C. 1967, 746; J. Chem. Soc. Perkin Trans. I, 1059 [1982]; Arzneimittel Forsch. 27, 343 [1977]; Compt. Rend. 253, 1974 [1961]; Bull. Soc. Chim. Fr. 1963, 144; French Patent FR-M-1559 vom 3.12.62). Man erhält die bekannten, ebenso wie die nicht bekannten Verbindungen der Formel (IV) in Analogie zu bekannten Verfahren (vgl. z. B. J. org. Chem. 51, 1719 [1986]; US-A-4 098 896 sowie die Herstellungsbeispiele). In 1-Stellung unsubstituierte Triazolinone der Formel (IVa),

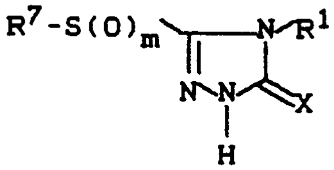

(IVa)

in welcher

$R^1$, $R^7$ und X die oben angegebene Bedeutung haben und

m für eine Zahl 1 oder 2 steht,

erhält man aus den entsprechenden Verbindungen der Formel (IVb),

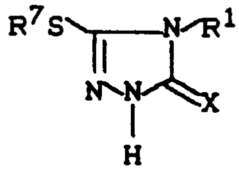

(IVb)

in welcher

$R^1$, $R^7$ und X die oben angegebene Bedeutung haben,

in allgemein bekannter Art und Weise mit üblichen Oxidationsmitteln, beispielsweise durch Umsetzung mit 3-Chlorperbenzoesäure gegebenenfalls in Gegenwart eines Verdünnungsmittels wie beispielsweise Dichlormethan oder Acetonitril und gegebenenfalls in Gegenwart eines Katalysators wie beispielsweise Ammoniummolybdat bei Temperaturen zwischen 0 °C und 40 °C.

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen $R^4$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate sind allgemein bekannte Verbindungen der organischen Chemie (vgl. z.B. Saul Patai, "The Chemistry of Cyanates and their Thioderivates" J. Wiley & Sons, New York 1977).

Als Verdünnungsmittel zur Durchführung des erfindungsgemäßen Verfahrens (a) kommen vorzugsweise inerte organische Lösungsmittel infrage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen wie Pyridin.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist auch möglich das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen O °C und + 150 °C, vorzugsweise bei Temperaturen zwischen + 10 °C und + 80 °C.

Das erfindungsgemäße Verfahren (a) wird üblicherweise unter Normaldruck durchgeführt. Es ist jedoch auch möglich unter erhöhtem Druck zu arbeiten.

Zur Durchführung des erfindungsgemäßen Verfahrens (a) setzt man pro Mol an 1-Chlor-(thio)carbonyltriazolinon der Formel (II) im allgemeinen 1,0 bis 5,0 Mol, vorzugsweise 1,0 bis 2,5 Mol am Amin der Formel (III) und gegebenenfalls 1,0 bis 2,5 Mol an Säurebindemittel ein. Die Reaktionsdurchführung, Aufarbeitung und Isolierung der Reaktionsprodukte erfolgt in Analogie zu allgemein bekannten Verfahren.

Als Verdünnungmittel zur Durchführung des erfindungsgemäßen Verahrens (b) kommen ebenfalls inerte organische Lösungsmittel infrage. Vorzugsweise verwendet man die bei Verfahren (a) genannten Verdünnungsmittel.

Das erfindungsgemäße Verfahren (b) kann gegebenenfalls in Gegenwart eines basischen Reaktionshilfmittels durchgeführt werden. Als solche kommen alle üblichen anorganischen und organischen Basen infrage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Der Zusatz solcher Katalysatoren ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und + 150 °C, vorzugsweise bei Temperaturen zwischen + 40 °C und + 120 °C.

Das erfindungsgemäße Verfahren (b) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (b) setzt man pro Mol an in 1-Stellung unsubstituiertem Triazolinon der Formel (IV) in allgemeinen 1,0 bis 5,0 Mol, vorzugsweise 1,0 bis 2,5 Mol an Iso-(thio)cyanat der Formel (V) und gegebenenfalls 1,0 bis 2,5 Mol an Reaktionshilfsmittel ein. Die Reaktionsdurchführung, Aufarbeitung und Isolierung der Reaktionsprodukte erfolgt in Analogie zu allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt, im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung mono- und dikotyler Unkräuter insbesondere in monokotylen Kulturen einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

In entsprechenden Aufandmengen zeigen die erfindungsgemäßen Wirkstoffe außerdem auch insektizide, bakterizide und fungizide Wirksamkeit und lassen sich beispielsweise zur Bekämpfung von Hygiene und Vorratsschädlingen oder zur Bekämpfung von Pilzkrankheiten im Getreide- und Reisanbau, wie beispielsweise gegen den Erreger des Getreidemehltaus (Erysiphe graminis) oder gegen den Erreger der Reisflekkenkrankheit (Pyricularia oryzae) einsetzen. In diesem Anwendungsbereich zeigen die erfindungsgemäßen Wirkstoffe neben guten protektiven auch systemische Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage; z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie

synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, infrage. Auch Mischungen mit N,N-Dimethyl-N′-(3-chlor-4-methylphenyl)-harnstoff;
N,N-Dimethyl-N′-(4-isopropylphenyl)-harnstoff;
2-Chlor-4-ethylamino-6-isopropylamino-1,3,5-triazin;
4-Ethylamino-2-t-butylamino-6-methylthio-s-triazin;
2-Chlor-4-ethylamino-6-(3-cyanopropylamino)-1,3,5-triazin;
4-Amino-6-t-butyl-3-ethylthio-1,2,4-triazin-5(4H)-on;
N-Methyl-2-(1,3-benzthiazol-2-yloxy)-acetanilid;
N,N-Diisopropyl-S-(2,3,3-trichlorallyl)-thiolcarbamat;
2-Ethyl-6-methyl-N-(1-methyl-2-methoxyethyl)-chloracetanilid;
Chloressigsäure-N-(methoxymethyl)-2,6-diethylanilid;
2-Chlor-N-(2,6-dimethylphenyl)-N-[(1H)-pyrazol-1-yl-methyl]-acetamid;
α-Chlor-2′,6′-diethyl-N-(2-propoxyethyl)-acetanilid;
2-[5-Methyl-5-(1-methylethyl)-4-oxo-2-imidazolin-2-yl]-3-chinolincarbonsäure;
Methyl-2-[4,5-dihydro-4-methyl-4-(1-methylethyl)-5-oxo-1H-imidazol-2-yl]-4(5)-methylbenzoat;
Methyl-5-(2,4-dichlorphenoxy)-2-nitrobenzoat;
5-(2-Chlor-4-trifluormethyl-phenoxy)-N-methylsulfonyl-2-nitrobenzamid;
(2-Ethoxy-1-methyl-2-oxo-ethyl)-5-[2-chlor-4-(trifluormethyl)-phenoxy]-2-nitrobenzoat;
5-(2-Chlor-4-trifluormethyl-phenoxy)-2-nitro-benzoesäure; 2,4-Dichlorphenoxyessigsäure;
2,4-Dichlorphenoxypropionsäure;
(2-Methyl-4-chlorphenoxy)-essigsäure;
(4-Chlor-2-methylphenoxy)-propionsäure;
3,5-Diiod-4-hydroxybenzonitril;
3,5-Dibrom-4-hydroxy-benzonitril;
2-Chlor-N-{[(4-methoxy-6-methyl-1,3,5-triazin-2-yl)-amino]-carbonyl}-benzolsulfonamid;
2-{[[((4-Methoxy-6-methyl-1,3,5-triazin-2-yl)-amino)-carbonyl]-amino]-sulfonyl}-benzoesäure oder deren Methylester
2-{-4-[(3-Chlor-5-(trifluormethyl)-2-pyridinyl)-oxy]-phenoxy}-propansäure bzw. -propansäureethylester;
2-[4-(3,5-Dichlorpyrid-2-yloxy)-phenoxy]-propionsäure-(trimethylsilylmethylester);
0-(6-Chlor-3-phenyl-pyridazin-4-yl)-S-octyl-thiocarbonat und 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch

Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können dabei ebenfalls in einem größeren Bereich variiert werden. Im allgemeinen verwendet man bei der Anwendung als Wachstumsregulatoren pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenenheiten richtet.

Herstellungsbeispiele:

Beispiel 1:

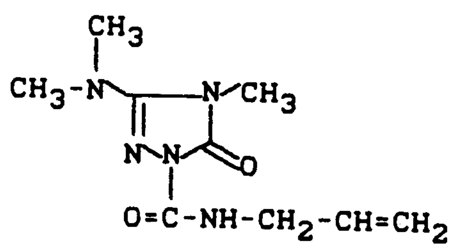

(Verfahren a)

Zu 10,25 g (0,05 Mol) 1-Chlorcarbonyl-3-dimethylamino-4-methyl-1,2,4-triazolin-5-on in 200 ml Acetonitril tropft man unter Rühren 5,7 g (0,1 Mol) Allylamin so, daß die Reaktionstemperatur 40 °C nicht übersteigt. Nach beendeter Zugabe rührt man vier Stunden bei Raumtemperatur, filtriert dann ausgefallenes Allylaminhydrochlorid ab, engt das Filtrat im Vakuum ein, nimmt den öligen Rückstand in 150 ml Dichlormethan auf, wäscht dreimal mit jeweils 50 ml Wasser, trocknet über Natriumsulfat und entfernt das Lösungsmittel in Vakuum.

Man erhält 8,8 g (79 % der Theorie) an 1-Allylaminocarbonyl-3-dimethylamino-4-methyl-1,2,4-triazolin-5-on als Öl

$^1$H-NMR ($CDCl_3$/TMS) $\delta$ = 4,0 (2H, $CH_2$); 5,8-6,0 (1H;CH=); 5,1-5,3 (2H; =$CH_2$) ppm.

Herstellung der Ausgangsverbindungen:

Beispiel II-1:

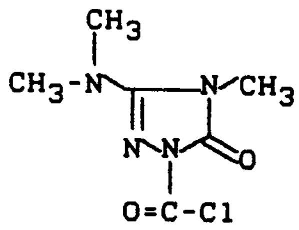

71 g (0,5 Mol) 3-Dimethylamino-4-methyl-1H-1,2,4-triazolin-5-on in 300 ml Toluol werden unter Einleiten von Phosgen auf 120 °C erwärmt. Insgesamt leitet man 115 g (1,15 Mol) Phosgen ein. Ab 80 °C findet eine lebhafte Chlorwasserstoffentwicklung statt. Nach beendeter Phosgen-Einleitung rührt man weitere 5 Stunden bei 120 °C, entfernt überschüssiges Phosgen und Chlorwasserstoff durch Ausblasen mit Stickstoff und filtriert die Mischung bei 20 °C. Das Filtrat wird mit 1 l Cyclohexan verrührt, das ausgefallene Produkt abgesaugt, mit Cyclohexan gewaschen und getrocknet.

Man erhält 70 g (69 % der Theorie) an 1-Chlorcarbonyl-3-dimethylamino-4-methyl-1,2,4-triazolin-5-on vom Schmelzpunkt 78 °C - 80 °C.

Beispiel IV-1:

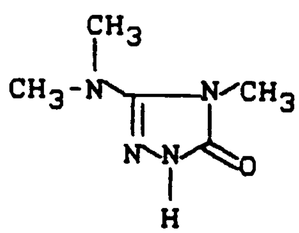

In eine Suspension aus 152,5 g (1 Mol) 1-Amino-2,2,3-trimethylguanidiniumhydrochlorid in 1000 ml Acetonitril leitet man innerhalb von 2 Stunden unter Rühren bei 80 °C 150 g (1,5 Mol) Phosgen, rührt 30 Minuten bei 80 °C nach, kühlt auf 20 °C ab, entfernt überschüssiges Phosgen durch Ausblasen mit Stickstoff, saugt ausgefallenes Produkt ab, löst es in 1000 ml Wasser, neutralisiert mit konzentrierter Natronlauge und engt im Vakuum zu Trockne ein. Der ölige Rückstand wird in 1000 ml Acetonitril aufgenommen, filtriert und das Filtrat im Vakuum vom Lösungsmittel befreit.

Man erhält 80 g (57 % der Theorie) an 3-Dimethylamino-4-methyl-1H-1,2,4-triazolin-5-on vom Schmelzpunkt 78 °C - 80 °C.

Beispiel VII-1:

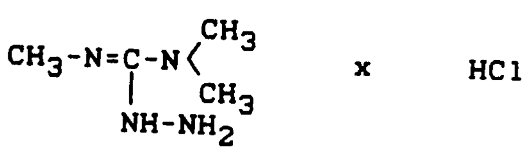

Zu 50 g (1 Mol) Hydrazinhydrat in 300 ml Isopropanol tropft man bei 20 °C bis 25 °C unter Rühren innerhalb von 30 Minuten eine Lösung von 78,5 g (0,5 Mol) Chlortrimethylformamidiniumhydrochlorid in 250

ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

Beispiel IX-1:

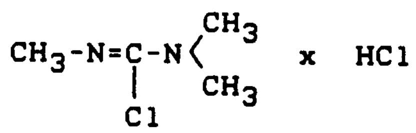

In eine Mischung aus 510 g (5 Mol) N,N,N'-trimethylharnstoff und 3 l Chlorbenzol leitet man bei 80 °C innerhalb 2,5 Stunden unter Rühren 545 g (5,5 Mol) Phosgen und rührt nach beendetem Einleiten weitere 45 Minuten bis zum Ende der Kohlendioxid-Entwicklung bei 80 °C nach. Die Reaktionsmischung wird abgekühlt auf 10 °C, das wasserempfindliche Produkt unter Stickstoff abgesaugt, mit 1 l Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76 °C bis 78 °C.

Beispiel 2:

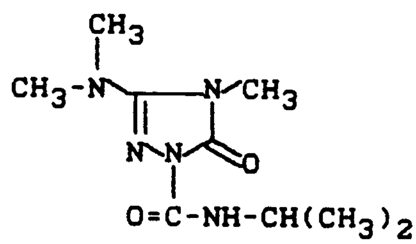

(Verfahren b)

7,1 g (0,05 Mol) 3-Dimethylamino-4-methyl-1H-1,2,4-triazolin-5-on in 100 ml Toluol werden mit 4,25 g (0,05 Mol) Isopropylisocyanat versetzt und 2 Stunden bei 120 °C gerüht. Das erkaltete Reaktionsgemisch wird filtriert und das Filtrat im Vakuum eingeengt.

Man erhält 9,8 g (87 % der Theorie) an 1-Isopropylaminocarbonyl-3-dimethylamino-4-methyl-1,2,4-triazolin-5-on vom Schmelzpunkt 36 °C - 38 °C.

Beispiel 3:

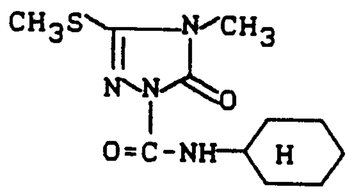

(Verfahren b)

Zu 1,5 g (0,01 Mol) 3-Methylthio-4-methyl-1H-1,2,4-triazolin-5-on (vgl. US-PS 4 098 896 bzw. US-PS 4 110 332) in 20 ml Dioxan gibt man 1 g (0,01 Mol) Triethylamin und 1,3 g (0,01 Mol) Cyclohexylisocyanat, rührt 12 Stunden bei 60 °C, engt im Vakuum zur Trockne ein, nimmt den Rückstand in 50 ml Dichlormethan auf, filtiert, wäscht das Filtrat zweimal mit jeweils 50 ml Wasser, trocknet über Natriumsulfat, entfernt das Lösungsmittel im Vakuum und verreibt den Rückstand mit Ether.

Man erhält 2,2 g (81 % der Theorie) an 1-Cyclohexylaminocarbonyl-3-methylthio-4-methyl-1,2,4-triazolin-5-on vom Schmelzpunkt 136 °C.

In entsprechender Weise und gemäß den allgemeinen Angaben zur Herstellung erhält man die folgenden substituierten Triazolinone der allgemeinen Formel (I):

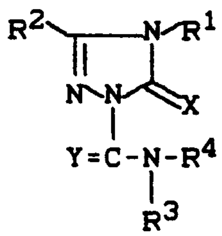

(I)

| Bsp. Nr. | $R^1$ | $R^2$ | $-N<^{R^3}_{R^4}$ | X | Y | physik. Eigenschaften |
|---|---|---|---|---|---|---|
| 4 | $CH_3$ | $-N(CH_3)_2$ | $-N(C_2H_5)_2$ | O | O | Fp 61-64° C |
| 5 | $CH_3$ | $-N(CH_3)_2$ | $-N<^{CH_3}_{C_6H_5}$ | O | O | Fp 76-77° C |
| 6 | $CH_3$ | $-N(CH_3)_2$ | $-N<^{CH_3}_{CH_2-CH_2-CN}$ | O | O | $^1$H-NMR*): 2,8-2,9; 3,6-3,8 |
| 7 | $CH_3$ | $-N(CH_3)_2$ | 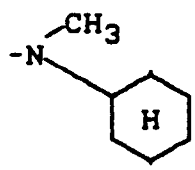 | O | O | Fp 82-83° C |
| 8 | $CH_3$ | $-N(CH_3)_2$ | 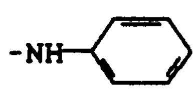 | O | O | Fp123-124° C |
| 9 | $CH_3$ | $-N(CH_3)_2$ | $-NH-CH_3$ | O | O | Fp 80-81° C |

| Bsp. Nr. | $R^1$ | $R^2$ | $-N\langle\begin{smallmatrix}R^3\\R^4\end{smallmatrix}$ | X | Y | physik. Eigenschaften |
|---|---|---|---|---|---|---|
| 10 | $CH_3$ | $-N(CH_3)_2$ | $-NH-C(CH_3)_3$ | O | O | $^1H$-NMR*): 1,4 |
| 11 | $CH_3$ | $-N(CH_3)_2$ | -NH-CO 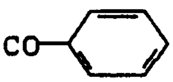 | O | O | Fp 183-184° C |
| 12 | $CH_3$ | $-N(CH_3)_2$ | -NH 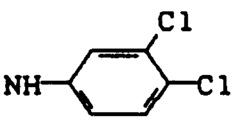 | O | O | Fp 159-160° C |
| 13 | $CH_3$ | $-N(CH_3)_2$ | $-N\langle\begin{smallmatrix}CH_2-CH_2-CN\\CH_2-CH_2-CN\end{smallmatrix}$ | O | O | Fp 149-151° C |
| 14 | $CH_3$ | $-N(CH_3)_2$ | -NH 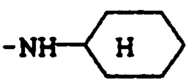 | O | O | Fp 91-93° C |
| 15 | $CH_3$ | $-N(CH_3)_2$ | $-NH-(CH_2)_5-CH_3$ | O | O | Fp 91-93° C |
| 16 | $CH_3$ | $-N(CH_3)_2$ | $-NH_2$ | O | O | Fp 161-162° C |
| 17 | $CH_3$ | $-N(CH_3)_2$ | $-NH-CH_2-$ 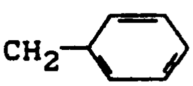 | O | O | Fp 65-67° C |
| 18 | $(CH_3)_2CH-$ | $-N(CH_3)_2$ | $-NH-C(CH_3)_3$ | O | O | $^1H$-NMR*): 1,5; 4,1-4,2 |

| Bsp. Nr. | $R^1$ | $R^2$ | 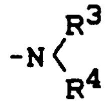 | X | Y | physik. Eigenschaften |
|---|---|---|---|---|---|---|
| 19 | 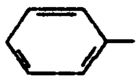 | $-N(CH_3)_2$ | $-NH-C(CH_3)_3$ | O | O | Fp 132-134° C |
| 20 | $CH_3$ | $-N(CH_3)_2$ | $-NH-CH_2-CF_3$ | O | O | Fp 78-80° C |
| 21 | $CH_3$ | $-N(CH_3)_2$ | 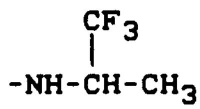 | O | O | Fp 101-103° C |
| 22 | $CH_3$ | $-N(CH_3)_2$ | 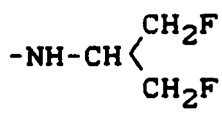 | O | O | Fp 79-81° C |
| 23 | $CH_3$ | $-N(CH_3)_2$ | 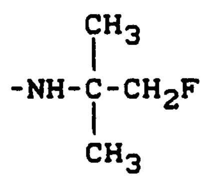 | O | O | Fp 84-86° C |
| 24 | $CH_3$ | $-N(CH_3)_2$ | 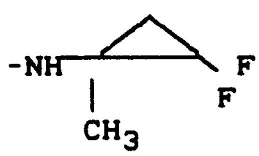 | O | O | $^1$H-NMR*): 1,5; 8,4 |
| 25 | $CH_3$ | 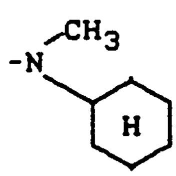 | $-NH-C(CH_3)_3$ | O | O | $^1$H-NMR*); 1,1-1,9; 3,0-3,1 |

| Bsp. Nr. | $R^1$ | $R^2$ | 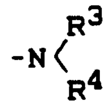 | X | Y | physik. Eigenschaften |
|---|---|---|---|---|---|---|
| 26 | $CH_3$ | $-N(CH_3)_2$ | 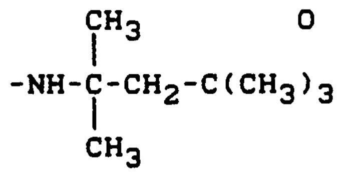 | O | O | Fp 75-76° C |
| 27 | $CH_3$ | 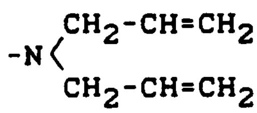 | $-NH-C(CH_3)_3$ | O | O | $^1H$-NMR*): 1,4; 5,8-5,9 |
| 28 | $CH_3$ | 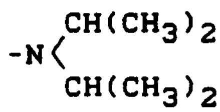 | $-NH-C(CH_3)_3$ | O | O | $^1H$-NMR*): 1,1; 1,4; 3,4-3,5 |
| 29 | $CH_3$ | $-N(C_2H_5)_2$ | $-NH-C(CH_3)_3$ | O | O | $^1H$-NMR*): 1,1-1,2; 1,4; 3,2 |
| 30 | $CH_3$ | 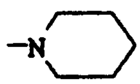 | $-NH-C(CH_3)_3$ | O | O | Fp 57-58° C |
| 31 | $CH_3$ | $-N(CH_3)_2$ | $-NH-(CH_2)_2-Cl$ | O | O | Fp 58-59° C |
| 32 | $CH_3$ | $-N(CH_3)_2$ | 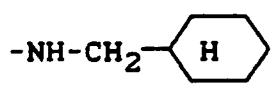 | O | O | $^1H$-NMR*): 0,9-1,8; 3,2 |

| Bsp. Nr. | $R^1$ | $R^2$ | $-N\langle^{R^3}_{R^4}$ | X | Y | physik. Eigenschaften |
|---|---|---|---|---|---|---|
| 33 | $CH_3$ | $-N(CH_3)_2$ | 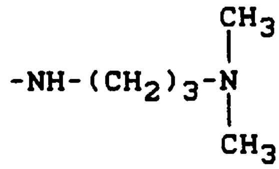 | O | O | $^1$H-NMR*): 1,7-1,8; 2,3-2,4; 3,4 |
| 34 | $CH_3$ | $-N(CH_3)_2$ | $-NH-(CH_2)_{11}-CH_3$ | O | O | Fp 46-48° C |
| 35 | $CH_3$ | $-N(CH_3)_2$ | 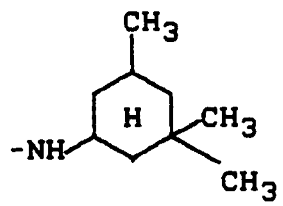 | O | O | $^1$H-NMR*): 0,9; 3,9 |
| 36 | $CH_3$ | $-N(CH_3)_2$ | $-NH-(CH_2)_2-OCH_3$ | O | O | $^1$H-NMR*): 3,4; 3,5-3,6 |
| 37 | $CH_3$ | $-N(CH_3)_2$ | 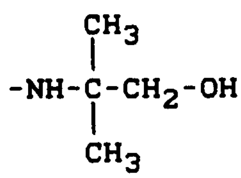 | O | O | Fp 108-110° C |
| 38 | $CH_3$ | $-N(CH_3)_2$ | 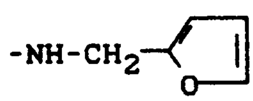 | O | O | Fp 115-117° C |
| 39 | $CH_3$ | $-N(CH_3)_2$ | $-NH-CH_2-CH(CH_3)_2$ | O | O | Fp 57-59° C |

| Bsp. Nr. | $R^1$ | $R^2$ | 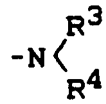 | X | Y | physik. Eigenschaften |
|---|---|---|---|---|---|---|
| 40 | $CH_3$ | $-N(CH_3)_2$ | $-NH-(CH_2)_3-$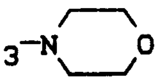 | O | O | $^{1}H$-NMR*): 2,45; 3,7 |
| 41 | $CH_3$ | $-N(CH_3)_2$ | -NH-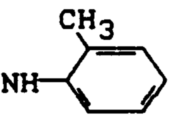 | O | O | Fp 126-128° C |
| 42 | $CH_3$ | $-N(CH_3)_2$ | 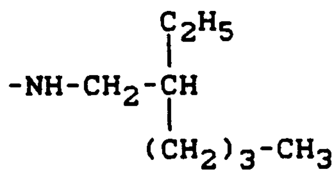 | O | O | $^{1}H$-NMR*): 0,9; 1,3-1,5; 3,45 |
| 43 | $CH_3$ | $-N(CH_3)_2$ | 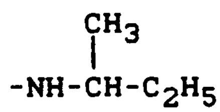 | O | O | $^{1}H$-NMR*): 1,2; 3,9-3,95 |
| 44 | $CH_3$ | $-N(CH_3)_2$ | -NH-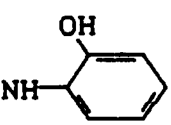 | O | O | Fp 191-192° C |
| 45 | $CH_3$ | $-N(CH_3)_2$ | -NH-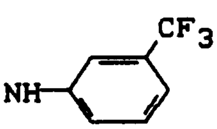 | O | O | Fp 94-96° C |
| 46 | $CH_3$ | $-N(CH_3)_2$ | -NH-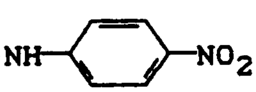 | O | O | Fp 236-238° C |

| Bsp. Nr. | $R^1$ | $R^2$ | $-N\langle^{R^3}_{R^4}$ | X | Y | physik. Eigenschaften |
|---|---|---|---|---|---|---|
| 47 | $CH_3$ | $-N(CH_3)_2$ | 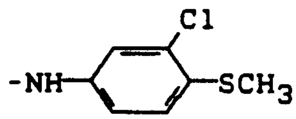 | O | O | Fp 172-174° C |
| 48 | $CH_3$ | $-N(CH_3)_2$ | 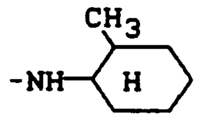 | O | O | $^1H$-NMR*): 0,9-1,9 |
| 49 | $CH_3$ | $-N(CH_3)_2$ | 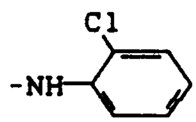 | O | O | Fp 139-140° C |
| 50 | $CH_3$ | $-N(CH_3)_2$ | 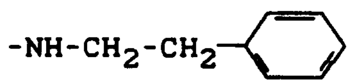 | O | O | $^1H$-NMR*): 7,1-7,3 |
| 51 | $CH_3$ | $-N(CH_3)_2$ | 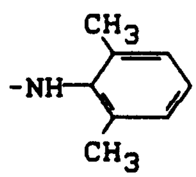 | O | O | Fp 136-138° C |
| 52 | $CH_3$ | $-N(CH_3)_2$ | 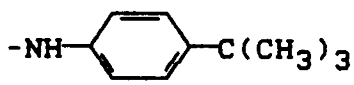 | O | O | Fp 126-128° C |
| 53 | $CH_3$ | $-N(CH_3)_2$ | $-NH-CH_2-C(CH_3)_3$ | O | O | Fp 72-73° C |

| Bsp. Nr. | $R^1$ | $R^2$ | $-N\langle{}^{R^3}_{R^4}$ | X | Y | physik. Eigenschaften |
|---|---|---|---|---|---|---|
| 54 | $CH_3$ | $-N(CH_3)_2$ | 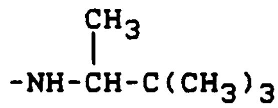 | O | O | Fp 73-74° C |
| 55 | $CH_3$ | $-N(CH_3)_2$ | 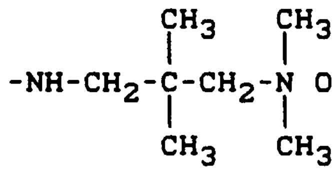 | O | O | $^1$H-NMR*): 2,2; 3,3 |
| 56 | $CH_3$ | $-N(CH_3)_2$ | 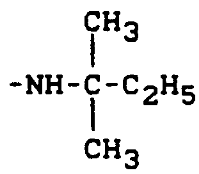 | O | O | Fp 81-83° C |
| 57 | $CH_3$ | $-N(CH_3)_2$ | 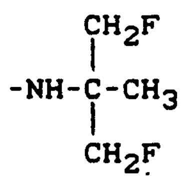 | O | O | Fp 96-97° C |
| 58 | $CH_3$ | $-N(CH_3)_2$ | 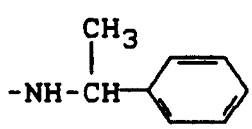 | O | O | $^1$H-NMR*): 7,2-7,3 |
| 59 | $CH_3$ | $-N(CH_3)_2$ | $-NH-(CH_2)_3-O-C_2H_5$ | O | O | $^1$H-NMR*): 3,4-3,5 |
| 60 | $CH_3$ | $-N(CH_3)_2$ | 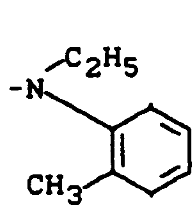 | O | O | Fp 99-100° C |

| Bsp. Nr. | $R^1$ | $R^2$ | $R^3$ -N⟨ $R^4$ | X | Y | physik. Eigenschaften |
|---|---|---|---|---|---|---|
| 61 | $CH_3$ | $-N(CH_3)_2$ | 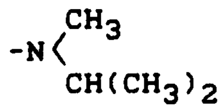 | O | O | $^1H$-NMR*): 3,4-3,5 |
| 62 | $CH_3$ | $-N(CH_3)_2$ | 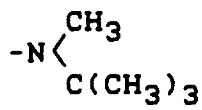 | O | O | $^1H$-NMR*): 1,4; 2,95 |
| 63 | $CH_3$ | $-N(CH_3)_2$ | 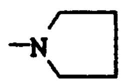 | O | O | $^1H$-NMR*): 1,9; 3,6-3,7 |
| 64 | $CH_3$ | $-N(CH_3)_2$ | 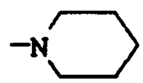 | O | O | $^1H$-NMR*): 1,6; 3,5 |
| 65 | $CH_3$ | $-N(CH_3)_2$ | 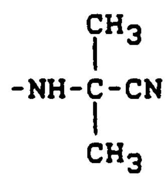 | O | O | Fp 147-150° C |
| 66 | $CH_3$ | $-N(CH_3)_2$ | 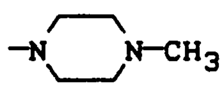 | O | O | $^1H$-NMR*); 2,3; 2,5; 3,6 |
| 67 | $CH_3$ | $-N(CH_3)_2$ | 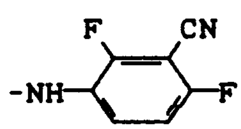 | O | O | Fp 215-217° C |

| Bsp. Nr. | $R^1$ | $R^2$ | $-N<^{R^3}_{R^4}$ | X | Y | physik. Eigenschaften |
|---|---|---|---|---|---|---|
| 68 | $CH_3$ | $-N(CH_3)_2$ | $-NH-(CH_2)_3-CH_3$ | O | O | $^1$H-NMR*): 3,35-3,45 |
| 69 | $CH_3$ | $-N(CH_3)_2$ | 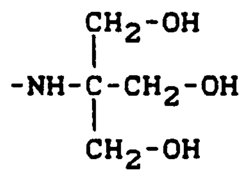 | O | O | Fp 163-165° C |
| 70 | $CH_3$ | $-N(CH_3)_2$ | 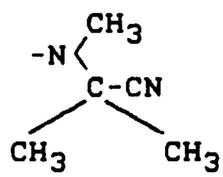 | O | O | Fp 117-119° C |
| 71 | $CH_3$ | $-N(CH_3)_2$ | 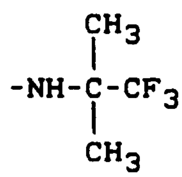 | O | O | Fp 57-59° C |
| 72 | $CH_3$ | $-N<^{CH_3}_{CH(CH_3)_2}$ | $-NH-C(CH_3)_3$ | O | O | $^1$H-NMR*): 3,5-3,6 |
| 73 | $CH_3$ | $-N(CH_3)_2$ | 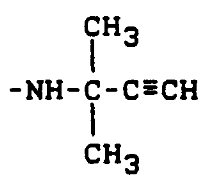 | O | O | Fp 115-117° C |

| Bsp. Nr. | $R^1$ | $R^2$ | $-N\langle^{R^3}_{R^4}$ | X | Y | physik. Eigenschaften |
|---|---|---|---|---|---|---|
| 74 | $CH_3$ | $-N\langle^{CH_3}_{C_2H_5}$ | $-NH-C(CH_3)_3$ | O | O | $^1H$-NMR*): 1,15-1,2; 3,5-3,6 |
| 75 | 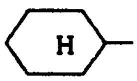 | $N(CH_3)_2$ | $-NH-C(CH_3)_3$ | O | O | Fp 108-110° C |
| 76 | $CH_3$ | $-N(CH_3)_2$ | 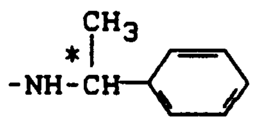 R (+) | O | O | Fp 80-82° C |
| 77 | $CH_3$ | $-N(CH_3)_2$ | 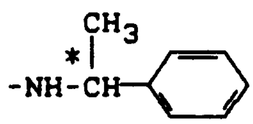 S (-) | O | O | Fp 48-50° C |
| 78 | $CH_3$ | $-N(CH_3)_2$ | 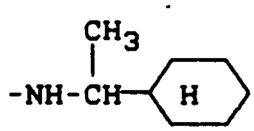 | O | O | $^1H$-NMR*): 3,85-3,95 |
| 79 | $CH_3$ | $-N(CH_3)_2$ | 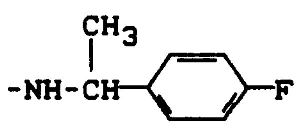 | O | O | $^1H$-NMR*): 7,0; 7,35 |
| 80 | $CH_3$ | $-N(CH_3)_2$ | 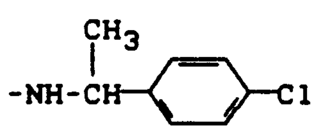 | O | O | $^1H$-NMR*): 7,3 |

| Bsp. Nr. | $R^1$ | $R^2$ | $-N{<}^{R^3}_{R^4}$ | X | Y | physik. Eigenschaften |
|---|---|---|---|---|---|---|
| 81 | $CH_3$ | $-N(CH_3)_2$ | 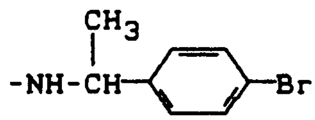 | O | O | $^1H$-NMR*): 7,25; 7,45 |
| 82 | $CH_3$ | $-N(CH_3)_2$ | 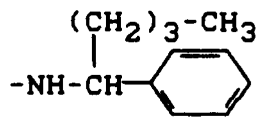 | O | O | $^1H$-NMR*): 7,3 |
| 83 | $CH_3$ | $-N(CH_3)_2$ | 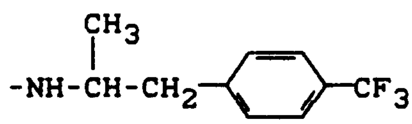 | O | O | $^1H$-NMR*): 4,3-4,4 |
| 84 | $CH_3$ | $-N(CH_3)_2$ | 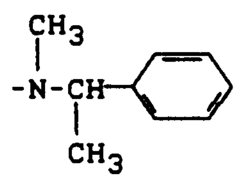 | O | O | $^1H$-NMR*): 5,6 |
| 85 | $CH_3$ | $-N(CH_3)_2$ | 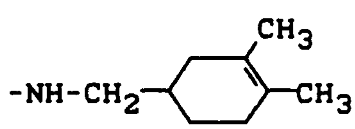 | O | O | $^1H$-NMR*): 3,3-3,35 |
| 86 | $CH_3$ | $-N(CH_3)_2$ | 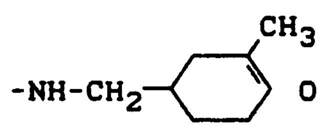 | O | O | $^1H$-NMR*): 3,3-3,35 |

| Bsp. Nr. | $R^1$ | $R^2$ | $-N{<}^{R^3}_{R^4}$ | X | Y | physik. Eigenschaften |
|---|---|---|---|---|---|---|
| 87 | $CH_3$ | $-N(CH_3)_2$ | 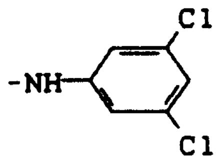 | O | O | Fp 181-182° C |
| 88 | $CH_3$ | $-N(CH_3)_2$ | 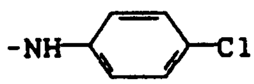 | O | O | Fp 162-163° C |
| 89 | $CH_3$ | $-N(CH_3)_2$ | 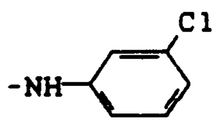 | O | O | Fp 103-105° C |
| 90 | $CH_3$ | $-N(CH_3)_2$ | 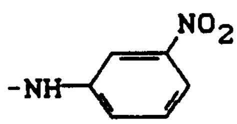 | O | O | Fp 187-188° C |
| 91 | $CH_3$ | $-N(CH_3)_2$ | 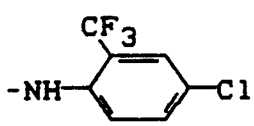 | O | O | Fp 138-139° C |
| 92 | $CH_3$ | $-N(CH_3)_2$ | 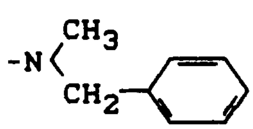 | O | O | $^1$H-NMR*): 7,1-7,4 |
| 93 | $CH_3$ | $-N(CH_3)_2$ | 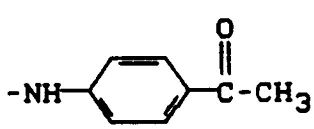 | O | O | Fp 181-182° C |

| Bsp. Nr. | $R^1$ | $R^2$ | 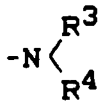 | X | Y | physik. Eigenschaften |
|---|---|---|---|---|---|---|
| 94 | $CH_3$ | $-N(CH_3)_2$ | 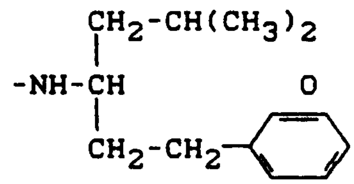 | O | O | $^1$H-NMR*): 4,05-4,15 |
| 95 | $CH_3$ | $-N(CH_3)_2$ | 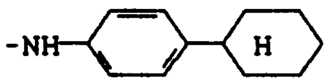 | O | O | Fp 141-142° C |
| 96 | $CH_3$ | $-N(CH_3)_2$ | 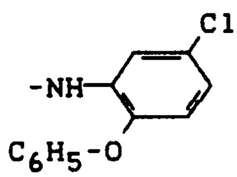 | O | O | Fp 137-138° C |
| 97 | $CH_3$ | $-N(CH_3)_2$ | 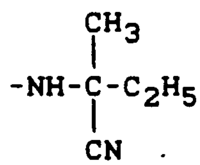 | O | O | $^1$H-NMR*): 1,75; 3,0-2,1 |
| 98 | $CH_3$ | $-N(CH_3)_2$ | 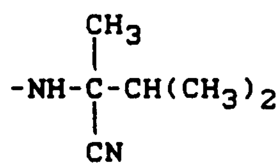 | O | O | $^1$H-NMR*): 1,1-1,2; 2,35 |
| 99 | $CH_3$ | $-N(CH_3)_2$ | 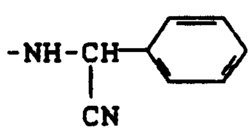 | O | O | Fp 117-119° C |

| Bsp. Nr. | $R^1$ | $R^2$ | $-N\langle^{R^3}_{R^4}$ | X | Y | physik. Eigenschaften |
|---|---|---|---|---|---|---|
| 100 | $CH_3$ | $-N(CH_3)_2$ | 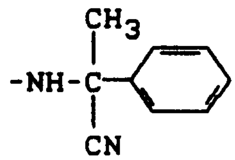 | O | O | $^1H$-NMR*): 7,3-7,6 |
| 101 | $CH_3$ | $-N(CH_3)_2$ | 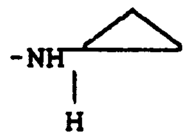 | O | O | Fp 92-93° C |
| 102 | $CH_3$ | $-N(CH_3)_2$ | $-NH-C_2H_5$ | O | O | Fp 49-51° C |
| 103 | $CH_3$ | $-N(CH_3)_2$ | $-NH-(CH_2)_2-CH_3$ | O | O | Fp 73-74° C |
| 104 | 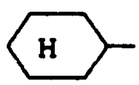 | $-N(CH_3)_2$ | 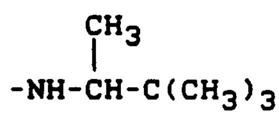 | O | O | $^1H$-NMR*): 3,15; 3,9 |
| 105 | $CH_3$ | $-N\langle^{CH_3}_{C_2H_5}$ | 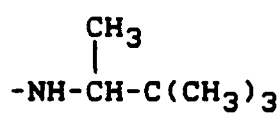 | O | O | $^1H$-NMR*): 3,15; 3,85 |
| 106 | $CH_3$ | $-N(CH_3)_2$ | 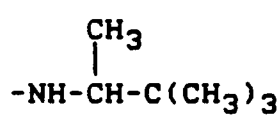 | O | S | Fp 91-92° C |
| 107 | $CH_3$ | $-N(CH_3)_2$ | $-NH-C(CH_3)_3$ | O | S | Fp 85-86° C |

| Bsp. Nr. | $R^1$ | $R^2$ | 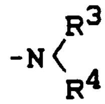 | X | Y | physik. Eigenschaften |
|---|---|---|---|---|---|---|
| 108 | $CH_3$ | $-N(CH_3)_2$ | $-NH-CH_2-CH=CH_2$ | O | S | Fp 87-89° C |
| 109 | $CH_3$ | $-N(CH_3)_2$ | $-NH-CH_2-CN$ | O | O | Fp 124-125° C |
| 110 | $CH_3$ | $-N(CH_3)_2$ | 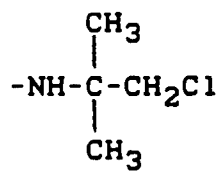 | O | O | Fp 70-72° C |
| 111 | $CH_3$ | $-N(CH_3)_2$ | 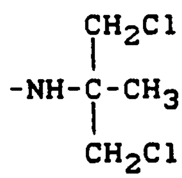 | O | O | Fp 113-115° C |
| 112 | $CH_3$ | $-N(CH_3)_2$ | 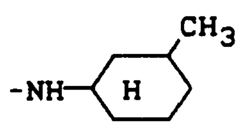 | O | O | Fp 60-61° C |
| 113 | $CH_3$ | $-N(CH_3)_2$ | 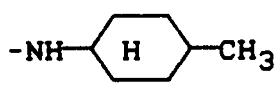 | O | O | $^1H$ -NMR*): 0,9; 7,8-8,2 |
| 114 | $CH_3$ | $-N(CH_3)_2$ | 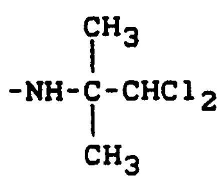 | O | O | Fp 120-121° C |

| Bsp. Nr. | $R^1$ | $R^2$ | 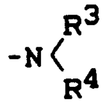 | X | Y | physik. Eigenschaften |
|---|---|---|---|---|---|---|
| 115 | $CH_3$ | $-N(CH_3)_2$ | 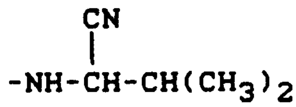 | O | O | Fp 72-73° C |
| 116 | $CH_3$ | $-N(CH_3)_2$ | 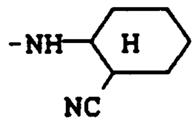 | O | O | Fp 78-80° C |
| 117 | $CH_3$ | $-N(CH_3)_2$ | 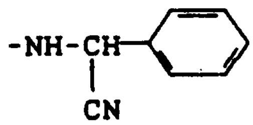 | O | O | $^1H$-NMR*): 2,95; 6,65 |
| 118 | $CH_3$ | 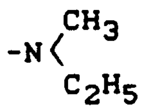 | $-NH-(CH_2)_3-CH_3$ | O | O | $^1H$-NMR*): 7,9 |
| 119 | $CH_3$ | $-N(CH_3)_2$ | 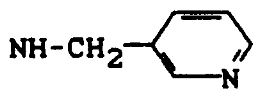 | O | O | $^1H$-NMR*): 7,3-8,5 |
| 120 | $CH_3$ | $-N(CH_3)_2$ | 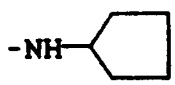 | O | O | Fp 75-77° C |
| 121 | $CH_3$ | $-N(CH_3)_2$ | 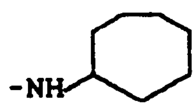 | O | O | Fp 58-59° C |
| 122 | $CH_3$ | $-N(CH_3)_2$ | 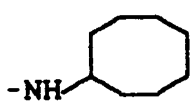 | O | O | Fp 47-48° C |

| Bsp. Nr. | $R^1$ | $R^2$ | $-N\langle^{R^3}_{R^4}$ | X | Y | physik. Eigenschaften |
|---|---|---|---|---|---|---|
| 123 | $CH_3$ | $-N(CH_3)_2$ | $-NH-CH(CH_3)-CH(CH_3)_2$ | O | O | $^1H$-NMR*): 3,85-3,95 |
| 124 | $CH_3$ | $-N(CH_3)_2$ | $-NH-CH_2-CH(CH_3)-C_2H_5$ | O | O | $^1H$-NMR*): 7,9 |
| 125 | $CH_3$ | $-N(CH_3)_2$ | $-NH-CH_2-CH_2-CH(CH_3)_2$ | O | O | $^1H$-NMR*): 7,8 |
| 126 | $CH_3$ | $-N(CH_3)_2$ | $-NH-CH(CH_3)-CH_2-CH(CH_3)_2$ | O | O | $^1H$-NMR*): 7,8 |
| 127 | $CH_3$ | $-N(CH_3)_2$ | $-NH-(CH_2)_3-C(CH_3)_3$ | O | O | $^1H$-NMR*): 7,9 |
| 128 | $CH_3$ | $-N(CH_3)_2$ | $-NH-CH_2-$ 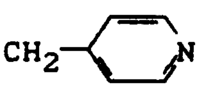 | O | O | $^1H$-NMR*): 7,2-8,5 |
| 129 | $CH_3$ | $-N(CH_3)_2$ | $-NH-CH(CN)-C(CH_3)_3$ | O | O | Fp 99-101° C |

| Bsp. Nr. | $R^1$ | $R^2$ | 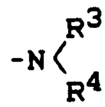 | X | Y | physik. Eigenschaften |
|---|---|---|---|---|---|---|
| 130 | $CH_3$ | $-N(CH_3)_2$ | 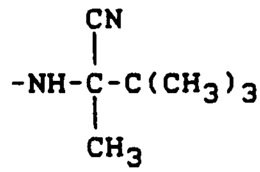 | O | O | Fp 167-168° C |
| 131 | $CH_3$ | $-N(CH_3)_2$ | 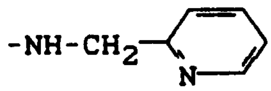 | O | O | Fp 109-111° C |
| 132 | $CH_3$ | $-N(CH_3)_2$ | 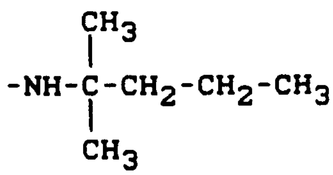 | O | O | Fp 30-31° C |
| 133 | $CH_3$ | $-N(CH_3)_2$ | $-NH-CH_2-CH_2-CN$ | O | O | Fp 117-119° C |
| 134 | $CH_3$ | $-N(CH_3)_2$ | 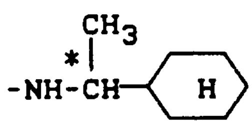 R(-) | O | O | $^1$H-NMR*): 3,9 |
| 135 | $CH_3$ | $-N(CH_3)_2$ | 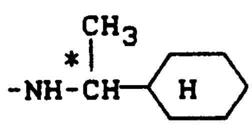 S(+) | O | O | $^1$H-NMR*): 3,9 |
| 136 | $CH_3$ | $-N(CH_3)_2$ | 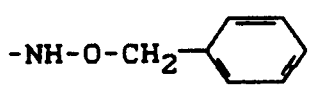 | O | O | Fp 130 °C |

| Bsp. Nr. | $R^1$ | $R^2$ | $-N\langle^{R^3}_{R^4}$ | X | Y | physik. Eigenschaften |
|---|---|---|---|---|---|---|
| 137 | $CH_3$ | $-N(CH_3)_2$ | $-NH-O-CH(CH_3)_2$ | O | O | Fp 103° C |
| 138 | $CH_3$ | $-N(CH_3)_2$ | $-NH-O-CH_2-CH(CH_3)_2$ | O | O | $^1H$-NMR*): 2,0; 3,8 |
| 139 | $CH_3$ | $-N(CH_3)_2$ | $-NH-O-CH_2-CH=CH_2$ | O | O | Fp 95 °C |
| 140 | $CH_3$ | $-N(CH_3)_2$ | $-NH-O-(CH_2)_2-CH_3$ | O | O | Fp 75 °C |
| 141 | $CH_3$ | $-N(CH_3)_2$ | 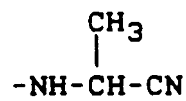 | O | O | Fp 102-104° C |
| 142 | $CH_3$ | $-SCH_3$ | 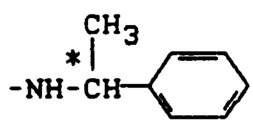 S(-) | O | O | Fp 99 °C |
| 143 | $CH_3$ | $-SCH_3$ | $-NH-CH(CH_3)_2$ | O | O | Fp 92 °C |
| 144 | $CH_3$ | $-SCH_3$ | $-NH-(CH_2)_2-OC_2H_5$ | O | O | Fp 59 °C |
| 145 | $CH_3$ | $-SCH_3$ | $-NH-CH_2-C(CH_3)_3$ | O | O | Fp 105 °C |

| Bsp. Nr. | $R^1$ | $R^2$ | $-N{<}^{R^3}_{R^4}$ | X | Y | physik. Eigenschaften |
|---|---|---|---|---|---|---|
| 146 | $CH_3$ | $-SCH_3$ | $-NH-C(CH_3)_3$ | O | O | Fp 108 °C |
| 147 | $CH_3$ | $-SCH_3$ | $-NH-CH_2-$ 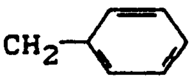 | O | O | Fp 131 °C |
| 148 | $CH_3$ | $-SCH_3$ | $-NH-\overset{CH_3}{\overset{\vert}{CH}}-C_2H_5$ | O | O | Fp 79 °C |
| 149 | $CH_3$ | $-SCH_3$ | $-NH-CH_2-CH(CH_3)_2$ | O | O | Fp 65 °C |
| 150 | $CH_3$ | $-SCH_3$ | $-NH-(CH_2)_5-CH_3$ | O | O | Fp 53 °C |
| 151 | $CH_3$ | $-SCH_3$ | $-NH-(CH_2)_2-OCH_3$ | O | O | Fp 99 °C |
| 152 | $CH_3$ | $-SCH_3$ | $-NH-(CH_2)_3-OCH_3$ | O | O | Fp 67 °C |
| 153 | $CH_3$ | $-SCH_3$ | $-NH-(CH_2)_2-CH_3$ | O | O | Fp 59 °C |
| 154 | $CH_3$ | $-SCH_3$ | 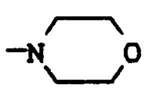 | O | O | Fp 111 °C |

| Bsp. Nr. | $R^1$ | $R^2$ | 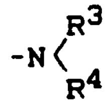 | X | Y | physik. Eigenschaften |
|---|---|---|---|---|---|---|
| 155 | $CH_3$ | $-SCH_3$ | 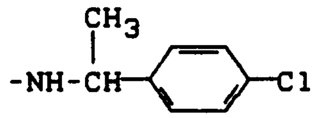 | O | O | Fp 63 °C |
| 156 | $CH_3$ | $-SCH_3$ | 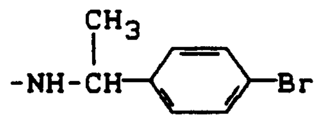 | O | O | Fp 112 °C |
| 157 | $CH_3$ | $-SCH_3$ | 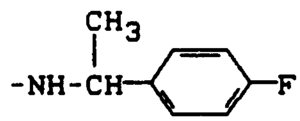 | O | O | Fp 115 °C |
| 158 | $CH_3$ | $-SCH_3$ | 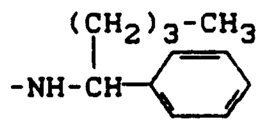 | O | O | Fp 78 °C |
| 159 | $CH_3$ | $-SCH_3$ | 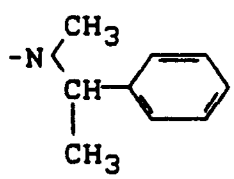 | O | O | $^1H$-NMR*): 1,7; 4,5 |
| 160 | $CH_3$ | $-SCH_3$ | 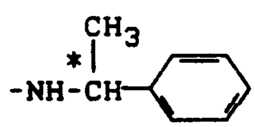 R(+) | O | O | Fp 105 °C |
| 161 | $CH_3$ | $-SCH_3$ | 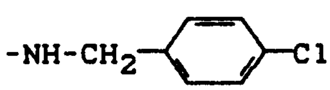 | O | O | Fp 217 °C |

| Bsp. Nr. | $R^1$ | $R^2$ | 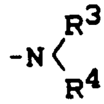 | X | Y | physik. Eigenschaften |
|---|---|---|---|---|---|---|
| 162 | $CH_3$ | 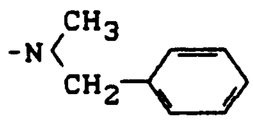 | $-NH-CH_3$ | O | O | Fp 118-119° C |
| 163 | $CH_3$ | $-N(CH_3)_2$ | 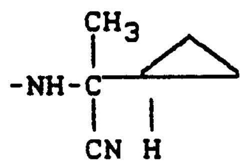 | O | O | Fp 204 °C (Zers.) |
| 164 | $CH_3$ | $-N(CH_3)_2$ | 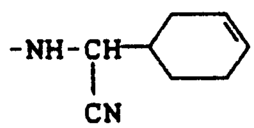 | O | O | Fp 118-120° C |
| 165 | $CH_3$ | $-N(CH_3)_2$ | 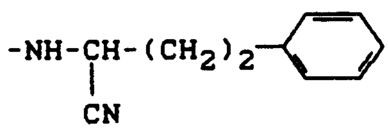 | O | O | Fp 110-112° C |
| 166 | $CH_3$ | $-N(CH_3)_2$ | 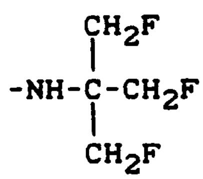 | O | O | Fp 110-112° C |
| 167 | $CH_3$ | $-N(CH_3)_2$ | 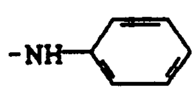 | S | O | Fp 144-145° C |
| 168 | $CH_3$ | $-N(CH_3)_2$ | 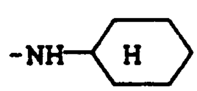 | S | O | Fp 90-91° C |

| Bsp. Nr. | $R^1$ | $R^2$ | 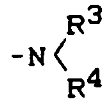 | X | Y | physik. Eigenschaften |
|---|---|---|---|---|---|---|
| 169 | $CH_3$ | $-N(CH_3)_2$ | 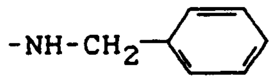 | S | O | Fp 67-68° C |
| 170 | $CH_3$ | $-N(CH_3)_2$ | 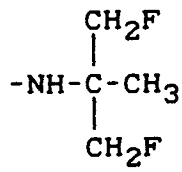 | S | O | Fp 99-100° C |
| 171 | $CH_3$ | $CH_3S$ | 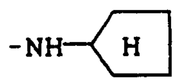 | O | O | Fp. 122° C |
| 172 | $CH_3$ | $CH_3S$ | 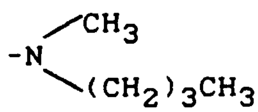 | O | O | $^1$H-NMR*): 3,1 (s) |
| 173 | $CH_3$ | $CH_3S$ | $-NH-(CH_2)_2-CH(CH_3)_2$ | O | O | $^1$H-NMR*): 0,9(dd) |
| 174 | $CH_3$ | $CH_3S$ | 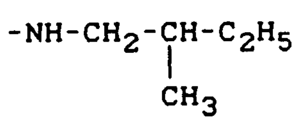 | O | O | $^1$H-NMR*): 1,7 (m) |
| 175 | $CH_3$ | $CH_3S$ | $-NH-(CH_2)_3-N(CH_3)_2$ | O | O | $^1$H-NMR*): 2,2 (s) |
| 176 | $CH_3$ | $CH_3S$ | $-NH-(CH_2)_4-CH_3$ | O | O | $^1$H-NMR*): 0,9 (t) |
| 177 | $CH_3$ | $CH_3S$ | 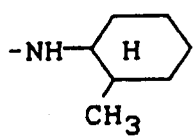 | O | O | Fp. 97° C |

| Bsp. Nr. | $R^1$ | $R^2$ | 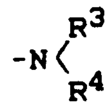 | X | Y | physik. Eigenschaften |
|---|---|---|---|---|---|---|
| 178 | $CH_3$ | $CH_3S$ | 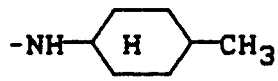 | O | O | Fp. 157° C |
| 179 | $CH_3$ | $CH_3S$ | $-NH-(CH_2)_2-Cl$ | O | O | Fp. 106° C |
| 180 | $CH_3$ | $CH_3S$ | $-NH-(CH_2)_2-$ 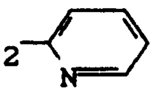 | O | O | Fp. 85° C |
| 181 | $CH_3$ | $CH_3S$ | 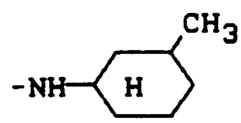 | O | O | $^1$H-NMR*): 3,8 (m) |
| 182 | $CH_3$ | $CH_3S$ | $-NH-(CH_2)_2-$ 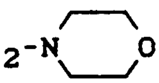 | O | O | Fp. 113° C |
| 183 | $CH_3$ | $CH_3S$ | 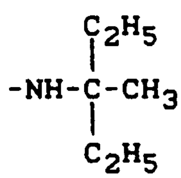 | O | O | Fp. 112° C |
| 184 | $CH_3$ | $CH_3S$ | $-NH-(CH_2)_2-C(CH_3)_3$ | O | O | Fp. 79° C |
| 185 | $CH_3$ | $CH_3S$ | 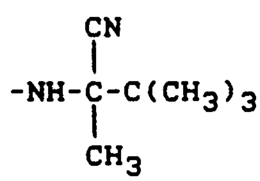 | O | O | Fp. 176° C |

| Bsp. Nr. | $R^1$ | $R^2$ | 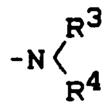 | X | Y | physik. Eigenschaften |
|---|---|---|---|---|---|---|
| 186 | $CH_3$ | $C_2H_5S$ | $-NH-CH(CH_3)_2$ | O | O | Fp. 110° C |
| 187 | $CH_3$ | $(CH_3)_2CHS-$ | $-NH-CH(CH_3)_2$ | O | O | Fp. 46° C |
| 188 | $CH_3$ | $CH_3S$ | 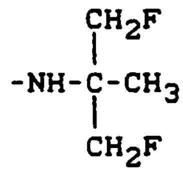 | O | O | Fp. 121° C |
| 189 | $CH_3$ | $CH_3S$ | 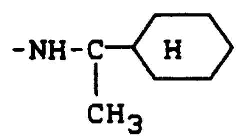 | O | O | Fp. 94° C |
| 190 | $CH_3$ | $CH_3S$ | 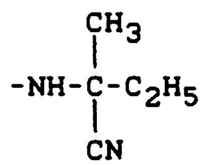 | O | O | Fp. 131° C |
| 191 | $C_2H_5$ | $CH_3S$ | $-NH-CH(CH_3)_2$ | O | O | Fp. 134° C |
| 192 | $CH_3$ | $CH_3S$ | 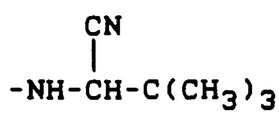 | O | O | Fp. 144° C |
| 193 | $CH_3$ | $CH_3S$ | 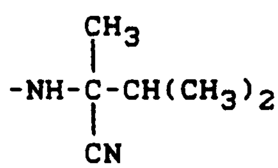 | O | O | Fp. 158° C |

| Bsp. Nr. | $R^1$ | $R^2$ | 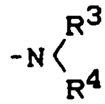 | X | Y | physik. Eigenschaften |
|---|---|---|---|---|---|---|
| 194 | $CH_3$ | $(CH_3)_2N-$ | 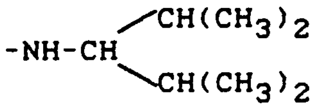 | O | O | Fp. 82-84° C |
| 195 | $CH_3$ | $(CH_3)_2N-$ | 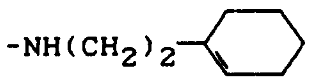 | O | O | Fp. 57-59° C |
| 196 | $CH_3$ | $(CH_3)_2N-$ | 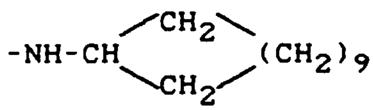 | O | O | Fp. 114° C-115° C |
| 197 | $CH_3$ | $(CH_3)_2N-$ | 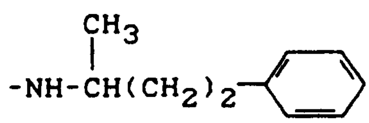 | O | O | $^1$H-NMR*): 1,25;1,85; 2,70;4,05; 7,1-7,25 |
| 198 | $CH_3$ | $(CH_3)_2N-$ | 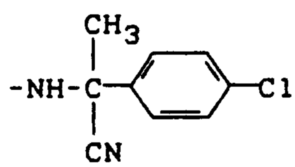 | O | O | Fp. 46-47° C |
| 199 | $CH_3$ | $CH_3S$ | 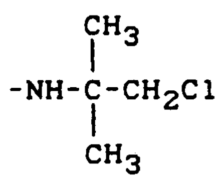 | O | O | Fp. 107° C-109° C |
| 200 | $CH_3$ | $CH_3S$ | $-NH-C(CH_3)_3$ | O | O | Fp. 119° C-120° C |
| 201 | $CH_3$ | $CH_3S$ | 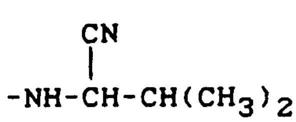 | O | O | Fp. 121° C-123° C |

| Bsp. Nr. | $R^1$ | $R^2$ | 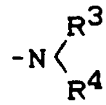 | X | Y | physik. Eigenschaften |
|---|---|---|---|---|---|---|
| 202 | $C_2H_5$ | $CH_3S$ | 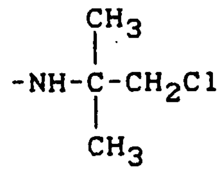 | O | O | Fp. 125° C-127° C |
| 203 | $C_2H_5$ | $CH_3S$ | 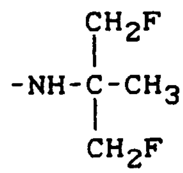 | O | O | Fp. 79-81° C |
| 204 | $C_2H_5$ | $CH_3S$ | 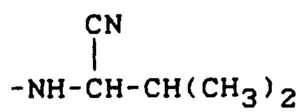 | O | O | Fp. 81-83° C |
| 205 | $C_2H_5$ | $CH_3S$ | 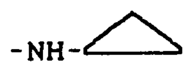 | O | O | Fp. 68-69° C |
| 206 | $C_2H_5$ | $CH_3S$ | 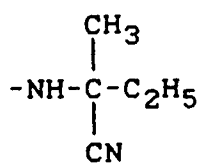 | O | O | Fp. 86-87° C |
| 207 | $C_2H_5$ | $CH_3S$ | 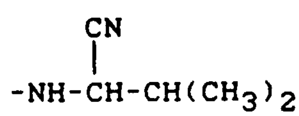 | O | O | Fp. 86-88° C |
| 208 | $C_2H_5$ | $CH_3S$ | 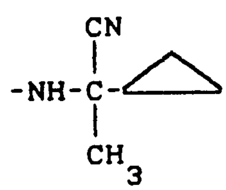 | O | O | Fp. 140° C-143° C |

| Bsp. Nr. | $R^1$ | $R^2$ | $-N\langle^{R^3}_{R^4}$ | X | Y | physik. Eigenschaften |
|---|---|---|---|---|---|---|
| 209 | $CH_3$ | $(CH_3)_2-N$ | $-NH-OCH_3$ | O | O | Fp. 109° C-112° C |
| 210 | $CH_3$ | $(CH_3)_2N-$ | $OCH_3$<br>$-NH-CH_2-CH$<br>$OCH_3$ | O | O | $^1$H-NMR*): 2,90; 3,50; 4,45 |
| 211 | $CH_3$ | $C_2H_5-S-$ | $-NH-CH_3$ | O | O | Fp. 137° C-139° C |
| 212 | $C_2H_5$ | $CH_3S$ | $-NH-CH_3$ | O | O | Fp. 159° C-160° C |
| 213 | $C_2H_5$ | $C_2H_5S-$ | $-NH-CH_3$ | O | O | Fp. 122° C-123° C |
| 214 | $C_2H_5$ | $-S-CH_2-$ 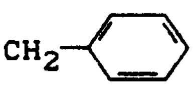 | $-NH-CH_3$ | O | O | Fp. 126° C-127° C |
| 215 | $CH_3$ | $-S-CH_2-CH=CH_2$ | $-NH-C(CH_3)_3$ | O | O | Fp. 126° C-127° C |
| 216 | $CH_3$ | $-S-C_2H_5$ | $NH-CH_2-C(CH_3)_3$ | O | O | Fp. 86-87° C |
| 217 | $CH_3$ | $-S-CH_2-$ 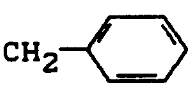 | $-NH-CH_2-C(CH_3)_3$ | O | O | Fp. 144° C-148° C |
| 218 | $C_2H_5$ | $-S-CH_3$ | $-NH-CH_2-C(CH_3)_3$ | O | O | Fp. 143° C-145° C |
| 219 | $C_2H_5$ | $-S-C_2H_5$ | $-NH-CH_2-C(CH_3)_3$ | O | O | Fp. 72-73° C |

| Bsp. Nr. | $R^1$ | $R^2$ | -N⟨ $R^3$ $R^4$ | X | Y | physik. Eigenschaften |
|---|---|---|---|---|---|---|
| 220 | $C_2H_5$ | -S-$CH_2$- 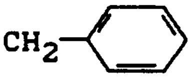 | $-NH-CH_2-C(CH_3)_3$ | O | O | Fp. 80-81° C |
| 221 | $CH_3$ | $-S-C_2H_5$ | -NH- 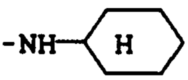 | O | O | Fp. 137° C-138° C |
| 222 | $CH_3$ | $-S-C_2H_5$ | -NH- 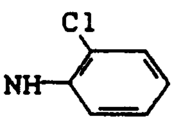 | O | O | Fp. 133° C-134° C |
| 223 | $C_2H_5$ | $-S-CH_3$ | -NH- 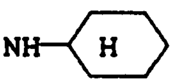 | O | O | Fp. 119° C-120° C |
| 224 | $C_2H_5$ | $-S-C_2H_5$ | -NH- 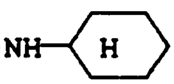 | O | O | Fp. 115° C-116° C |
| 225 | $C_2H_5$ | -S-$CH_2$- 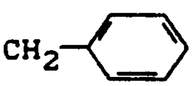 | -NH- 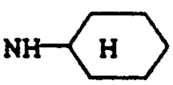 | O | O | Fp. 95-96° C |
| 226 | $CH_3$ | -S-$CH_2$- 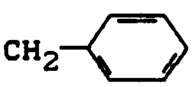 | -NH- 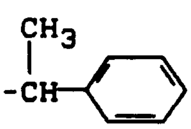 | O | O | Fp. 112° C-113° C |
| 227 | $C_2H_5$ | $-S-CH_3$ | -NH- 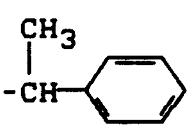 | O | O | Fp. 99-100° C |

| Bsp. Nr. | $R^1$ | $R^2$ | $-N\langle^{R^3}_{R^4}$ | X | Y | physik. Eigenschaften |
|---|---|---|---|---|---|---|
| 228 | $C_2H_5$ | $-S-C_2H_5$ | 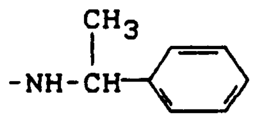 | O | O | Fp. 84-85° C |
| 229 | $CH_3$ | 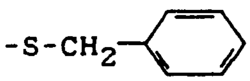 | $-NH-CH_3$ | O | O | Fp. 125° C-127° C |
| 230 | $CH_3$ | $-S-C_2H_5$ | 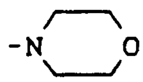 | O | O | $^1$H-NMR*): 3,17 (s) |
| 231 | $C_2H_5$ | $-S-C_2H_5$ | 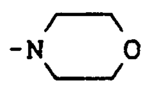 | O | O | $^1$H-NMR*): 3,20 (q); 1,43 (t); 1,22 (t) |
| 232 | $CH_3$ | $-S-C_2H_5$ | 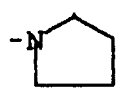 | O | O | $^1$H-NMR*): 3,18 (s); 1,43 (t) |
| 233 | $CH_3$ | $-S-C_2H_5$ | 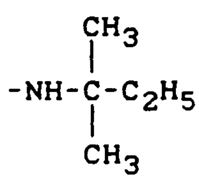 | O | O | Fp. 100° C-101° C |
| 234 | $C_2H_5$ | $-S-CH_2-CH=CH_2$ | $-NH-C(CH_3)_3$ | O | O | Fp. 75-77° C |
| 235 | $C_2H_5$ | $-S-CH_2-CH=CH_2$ | $-NH-CH_3$ | O | O | Fp. 84-86° C |
| 236 | $CH_3$ | $-S-CH_2-CH=CH_2$ | 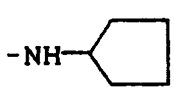 | O | O | Fp. 95-97° C |

| Bsp. Nr. | $R^1$ | $R^2$ | $-N\langle^{R^3}_{R^4}$ | X | Y | physik. Eigenschaften |
|---|---|---|---|---|---|---|
| 237 | $CH_3$ | $-S-CH_2-CH=CH_2$ | $-NH-CH_2-C(CH_3)_3$ | O | O | Fp. 84-86° C |
| 238 | $CH_3$ | $-S-CH_2-CH=CH_2$ | -NH 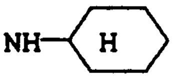 | O | O | Fp. 154° C-155° C |
| 239 | $CH_3$ | $-S-CH_2-CH=CH_2$ | -NH 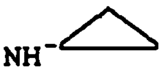 | O | O | Fp. 86-87° C |
| 240 | $CH_3$ | $-S-CH_2-CH=CH_2$ | $-NH-CH_2-CH=CH_2$ | O | O | $^1H$-NMR*): 3,22 (s); 3,85 (d) |
| 241 | $CH_3$ | $-S-CH_2-CH=CH_2$ | -NH 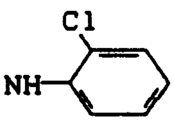 | O | O | Fp. 132° C-134° C |
| 242 | $-CH_2$ 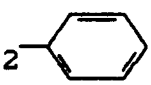 | $-S-CH_3$ | -NH 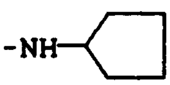 | O | O | $^1H$-NMR*): 2,60 (s); 4,77 (s) |
| 243 | $C_2H_5$ | $-S-C_2H_5$ | 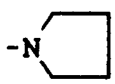 | O | O | $^1H$-NMR*): 1,27 (t); 1,42 (t); 3,19 (q); |
| 244 | $CH_3$ | $-S-CH_2-CH=CH_2$ | $-N(CH_2-CH=CH_2)_2$ | O | O | $^1H$-NMR*): 3,20 (s); 3,78 (d); 4,00 (d) |

| Bsp. Nr. | $R^1$ | $R^2$ | $-N(R^3)(R^4)$ | X | Y | physik. Eigenschaften |
|---|---|---|---|---|---|---|
| 245 | $C_2H_5$ | $-S-CH_2-CH=CH_2$ | $-NH-CH_2-C(CH_3)_3$ | 0 | 0 | $^1$H-NMR*): 3,90 (d); 3,72 (q); 3,20 (d); 1,30 (t) |
| 246 | $C_2H_5$ | $-S-CH_2-CH=CH_2$ | -NH 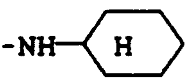 | 0 | 0 | Fp. 132° C-134° C |
| 247 | $C_2H_5$ | $-S-CH_2-CH=CH_2$ | -NH- 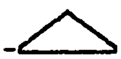 | 0 | 0 | $^1$H-NMR*): 3,89 (d); 3,68 (q); 2,80 (m); 1,28 (t) |
| 248 | $CH_3$ | $-S-CH_2-CH=CH_2$ | 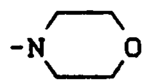 | 0 | 0 | $^1$H-NMR*): 3,19 (s); 3,84 (d) |
| 249 | $CH_3$ | $-S-CH_2-CH=CH_2$ | 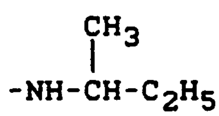 | 0 | 0 | Fp. 84-85° C |
| 250 | $CH_3$ | $-S-CH_2-CH=CH_2$ | 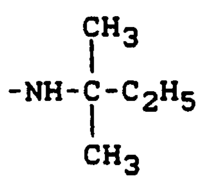 | 0 | 0 | Fp. 87-89° C |
| 251 | $-CH_2$ 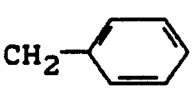 | $-SCH_3$ | -NH 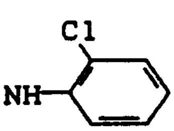 | 0 | 0 | Fp. 156° C-158° C |

| Bsp. Nr. | $R^1$ | $R^2$ | 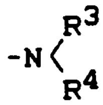 | X | Y | physik. Eigenschaften |
|---|---|---|---|---|---|---|
| 252 | $CH_3$ | -S-$CH_2$- 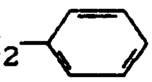 | 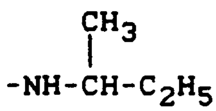 | O | O | Fp. 125° C-127° C |
| 253 | $CH_2$ | -S-$CH_2$- 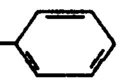 | 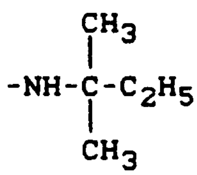 | O | O | Fp. 145° C-147° C |
| 254 | $CH_3$ | -S-$CH_2$- 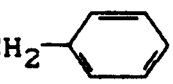 | -NH-$CH_2$-CH=$CH_2$ | O | O | Fp: 93-94° C |
| 255 | $CH_3$ | -S-$CH_2$- 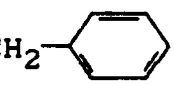 | -N($CH_2$=CH-$CH_2$)$_2$ | O | O | $^1$H-NMR*): 3,07 (s); 4,33 (s) |
| 256 | $CH_3$ | -S-$CH_2$- 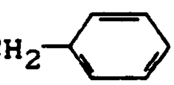 | 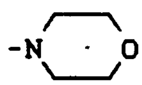 | O | O | Fp. 113° C-115° C |
| 257 | $CH_3$ | -S-$CH_2$- 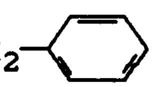 | -NH 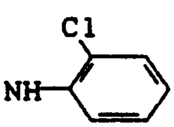 | O | O | Fp. 163° C-165° C |
| 258 | $C_2H_5$ | -S-CH($CH_3$)$_2$ | -NH-$CH_3$ | O | O | Fp. 82-84° C |
| 259 | $CH_3$ | -S-$C_2H_5$ | 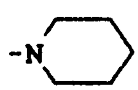 | O | O | $^1$H-NMR*): 3,16 (s); 1,42 (t) |

| Bsp. Nr. | $R^1$ | $R^2$ | $-N\langle^{R^3}_{R^4}$ | X | Y | physik. Eigenschaften |
|---|---|---|---|---|---|---|
| 260 | $C_2H_5$ | $-S-CH(CH_3)_2$ | $-NH-C(CH_3)_3$ | O | O | Fp. 78-79° C |
| 261 | $C_2H_5$ | $-S-CH(CH_3)_2$ | $-NH-CH_2-C(CH_3)_3$ | O | O | Fp. 61-62° C |
| 262 | $C_2H_5$ | $-S-CH(CH_3)_2$ | -NH- 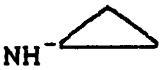 | O | O | Fp. 88-89° C |
| 263 | $C_2H_5$ | $-S-CH(CH_3)_2$ | -NH- 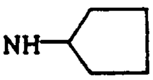 | O | O | Fp. 48-50° C |
| 264 | $C_2H_5$ | $-S-CH(CH_3)_2$ | -NH- 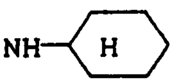 | O | O | Fp. 53-55° C |
| 265 | $C_2H_5$ | $-S-CH(CH_3)_2$ | -NH- 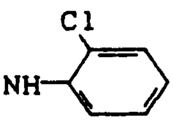 | O | O | Fp. 120° C-122° C |
| 266 | $C_2H_5$ | $-S-CH(CH_3)_2$ | -NH- 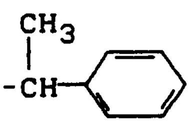 | O | O | Fp. 72-75° C |
| 267 | 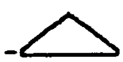 | $-S-CH_3$ | $-NH-C(CH_3)_3$ | O | O | Fp. 174° C-175° C |
| 268 | 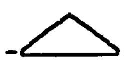 | $-S-CH_3$ | $-NH-CH_2-C(CH_3)_3$ | O | O | Fp. 111° C-113° C |

| Bsp. Nr. | $R^1$ | $R^2$ | $-N\langle^{R^3}_{R^4}$ | X | Y | physik. Eigenschaften |
|---|---|---|---|---|---|---|
| 269 | 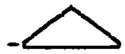 | $-S-CH_3$ | 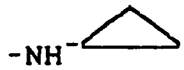 | O | O | Fp. 113° C-114° C |
| 270 | 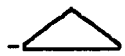 | $-S-CH_3$ | 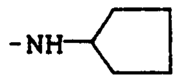 | O | O | Fp. 135° C-137° C |
| 271 | 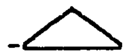 | $-S-CH_3$ | 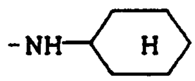 | O | O | Fp. 107° C-108° C |
| 272 | 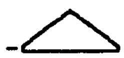 | $-S-CH_3$ | 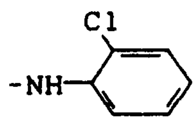 | O | O | Fp. 152° C-154° C |
| 273 | 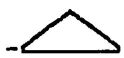 | $-S-CH_3$ | 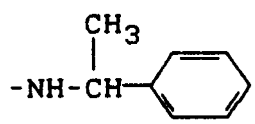 | O | O | Fp. 124° C-125° C |
| 274 | 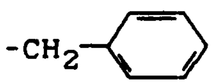 | $-S-CH_3$ | $-NH-CH_3$ | O | O | Fp. 154° C-156° C |
| 275 | $C_2H_5$ | $-SC_2H_5$ | 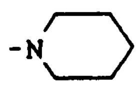 | O | O | $^1$H-NMR*): 1,26 (t); 3,65 (q); 1,42 (t); 3,19 (q); |
| 276 | 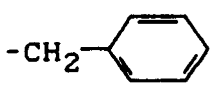 | $-S-CH_3$ | 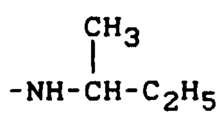 | O | O | Fp. 60-62° C |

| Bsp. Nr. | $R^1$ | $R^2$ | $-N\genfrac{}{}{0pt}{}{R^3}{R^4}$ | X | Y | physik. Eigenschaften |
|---|---|---|---|---|---|---|
| 277 | $-CH_2-$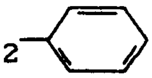 | $-S-CH_3$ | $-NH-C(CH_3)_3$ | O | O | Fp. 67-70° C |
| 278 | $-CH_2-$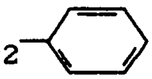 | $-S-CH_3$ | $-NH-CH_2-C(CH_3)_3$ | O | O | Fp. 79-81° C |
| 279 | $-CH_2-$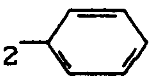 | $-S-CH_3$ | $CH_3$<br>$-NH-C-C_2H_5$<br>$CH_3$ | O | O | Fp. 68-71° C |
| 280 | $-CH_2-$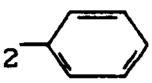 | $-S-CH_3$ | $-NH-CH_2-CH=CH_2$ | O | O | Fp. 80-82° C |
| 281 | $-CH_2-$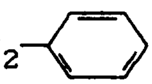 | $-S-CH_3$ | $-N(CH_2-CH=CH_2)_2$ | O | O | $^1$H-NMR*): 2,51 (s); 4,74 (s) |
| 282 | $-CH_2-$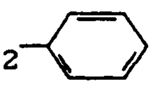 | $-S-CH_3$ | -NH-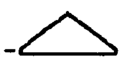 | O | O | Fp. 117° C-118° C |
| 283 | $-CH_2-$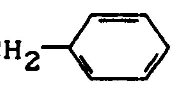 | $-S-CH_3$ | -NH-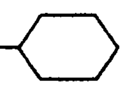 | O | O | Fp. 89-91° C |
| 284 | $-CH_2-$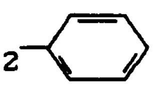 | $-S-CH_3$ | 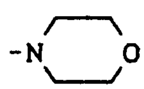 | O | O | Fp. 120° C-121° C |

| Bsp. Nr. | $R^1$ | $R^2$ | $-N\langle^{R^3}_{R^4}$ | X | Y | physik. Eigenschaften |
|---|---|---|---|---|---|---|
| 285 | $CH_3$ | 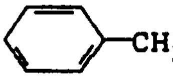 $-CH_2-S-$ | -NH 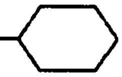 | O | O | Fp. 178° C-179° C |
| 286 | $CH_3$ | $(CH_3)_2CH-S-$ | -NH 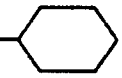 | O | O | Fp. 63-69° C |
| 287 | $CH_3$ | $(CH_3)_2CH-S-$ | $-NH-C(CH_3)_3$ | O | O | Fp. 86-87° C |
| 288 | $CH_3$ | $(CH_3)_2CH-S-$ | -NH 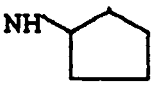 | O | O | Fp. 59-61° C |
| 289 | $CH_3$ | $(CH_3)_2CH-S-$ | -NH- 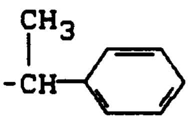 | O | O | Fp. 62-64° C |
| 290 | $CH_3$ | $(CH_3)_2CH-S-$ | $-NH-CH_2-C(CH_3)_3$ | O | O | Fp. 72-74° C |
| 291 | $CH_3$ | $(CH_3)_2-S-$ | -NH- 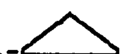 | O | O | Fp. 93-94° C |
| 292 | $CH_3$ | $C_2H_5-S-$ | -NH- 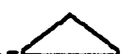 | O | O | Fp. 112° C-113° C |
| 293 | $CH_3$ | 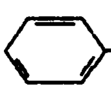 $-CH_2-S-$ | -NH- 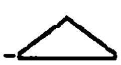 | O | O | Fp. 141° C-143° C |

| Bsp. Nr. | $R^1$ | $R^2$ | $-N\langle^{R^3}_{R^4}$ | X | Y | physik. Eigenschaften |
|---|---|---|---|---|---|---|
| 294 | $C_2H_5$ | $C_2H_5-S-$ | -NH-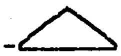 | O | O | Fp. 84-85° C |
| 295 | $C_2H_5$ | 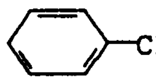$-CH_2-S-$ | -NH-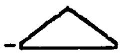 | O | O | Fp. 130° C-131° C |
| 296 | $CH_3$ | $C_2H_5-S-$ | $-NH-C(CH_3)_3$ | O | O | Fp. 123° C-124° C |
| 297 | $CH_3$ | 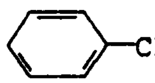$-CH_2-S-$ | $-NH-C(CH_3)_3$ | O | O | Fp. 176° C-177° C |
| 298 | $C_2H_5$ | $CH_3-S-$ | $-NH-C(CH_3)_3$ | O | O | Fp. 111° C-112° C |
| 299 | $C_2H_5$ | $C_2H_5-S-$ | $-NH-C(CH_3)_3$ | O | O | Fp. 85-87° C |
| 300 | $C_2H_5$ | 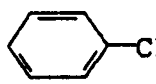$-CH_2-S-$ | $-NH-C(CH_3)_3$ | O | O | Fp. 151° C-152° C |
| 301 | $CH_3$ | $C_2H_5-S-$ | -NH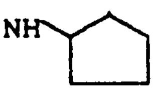 | O | O | Fp. 114° C-116° C |
| 302 | $CH_3$ | 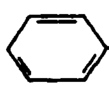$-CH_2-S-$ | -NH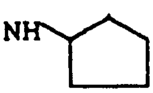 | O | O | Fp. 154° C-155° C |
| 303 | $C_2H_5$ | $CH_3-S$ | -NH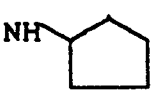 | O | O | Fp. 93-94° C |

| Bsp. Nr. | $R^1$ | $R^2$ | $-N(R^3)(R^4)$ | X | Y | physik. Eigenschaften |
|---|---|---|---|---|---|---|
| 304 | $C_2H_5$ | $C_2H_5$-S- | -NH 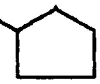 | O | O | Fp. 74-75° C |
| 305 | $C_2H_5$ | 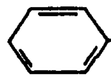 $CH_2$-S- | -NH 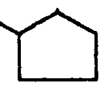 | O | O | Fp.75-77° C |
| 306 | $CH_3$ | $C_2H_5$-S- | $CH_3$ -NH-CH 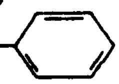 | O | O | Fp. 105° C-106° C |
| 307 | $C_2H_5$ | 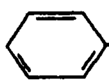 $CH_2$-S- | $CH_3$ -NH-CH 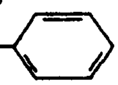 | O | O | Fp.88-89° C |
| 308 | $C_2H_5$ | $CH_2$=CH-$CH_2$-S- | -NH 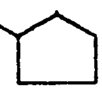 | O | O | Harz $^1$H-NMR*): 3,84 (d); 7,51 (d) |
| 309 | $C_2H_5$ | $CH_2$=CH-$CH_2$-S- | $CH_3$ -NH-CH 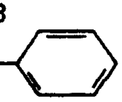 | O | O | Öl $^1$H-NMR*): 1,56 (d) 8,33 (d) 3,82 (d) |
| 310 | $CH_3$ | $CH_2$=CH-$CH_2$-S- | $CH_3$ -NH-CH 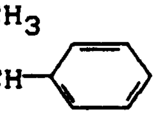 | O | O | Öl $^1$H-NMR*): 1,57 (d) 8,32 (d) 3,17 (s) 3,81 (d) |

| Bsp. Nr. | $R^1$ | $R^2$ | $-N\langle{}^{R^3}_{R^4}$ | X | Y | physik. Eigenschaften |
|---|---|---|---|---|---|---|
| 311 | $CH_3$ | $CH_2{=}CH{-}CH_2{-}S{-}$ | $-NH-CH_3$ | O | O | Fp. 95-97° C |
| 312 | 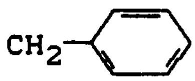 | $CH_3-S$ | 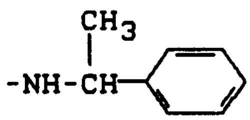 | O | O | Harz $^1$H-NMR*): 1,57 (d); 2,55 (s); 4,73 (s) |
| 313 | $CH_3$ | $C_2H_5-S-$ | 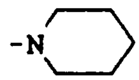 | O | O | Fp. 74-75° C |
| 314 | $C_2H_5$ | $C_2H_5-S-$ | 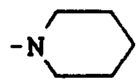 | O | O | 53-55° C |

*) Die $^1$H-NMR-Spektren wurden in $CDCl_3$ mit Tetramethylsilan (TMS) als innerem Standard aufgenommen. Angegebene ist die chemische Verschiebung als δ-Wert in ppm.

Anwendungsbeispiele:

In den folgenden Anwendungsbeispielen wurden die nachstehend aufgeführten Verbindungen als Vergleichsubstanzen eingesetzt:

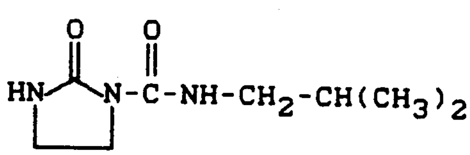

(A)

Imidazolidin-2-on-1-carbonsäureisobutylamid
(bekannt aus K. H. Büchel, "Pflanzenschutz und Schädlingsbekämpfung" S. 170, Thieme Verlag Stuttgart 1977)

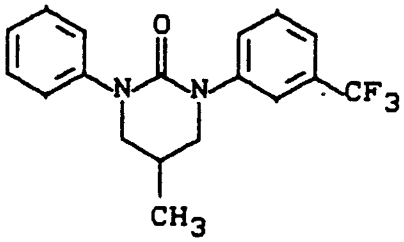

(B)

1-Phenyl-3-(3-trifluormethylphenyl)-5-methylperhydropryimidin-2-on
(bekannt aus EP-A-58 868)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß der Herstellungsbeispiele 2, 10, 14, 39, 43, 48, 53, 54, 56, 57, 58, 65, 68 und 71.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß der Herstellungsbeispiele 1,2,10, 14, 17, 32, 34, 36, 37, 39, 43, 50, 53, 54, 56, 57, 58, 65 und 68.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

Eine deutliche Überlegenheit in der Wirksamkeit im Vergleich zur unbehandelten Kontrolle zeigen in diesem Test beispielsweise die Verbindungen gemäß der Herstellungsbeispiele 15, 17, 32, 43, 48, 54, 56, 57 und 58.

**Patentansprüche**

**1.** Substituierte Triazolinone der allgemeinen Formel (I),

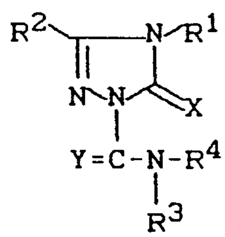

(I)

in welcher

$R^1$ für Methyl, Ethyl, n- oder i-Propyl, n-, i-, s oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,

$R^2$ für einen Rest

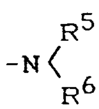

oder für einen Rest $S(O)_n$-$R^7$ steht,

$R^3$ und $R^4$ unabhängig voneinander jeweils für Wasserstoff, Methyl, Ethyl, n- oder i-Propyl, n-, i-, s-oder t-Butyl, n- oder i-Pentyl, n - oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n-oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden durch Fluor,Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen; außerdem für im Heterocyclylteil gegebenenfalls ein- bis dreifach, gleich oder verschieden durch Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl mit den Heterocyclen

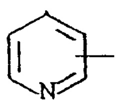 ; 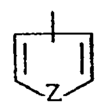 ; 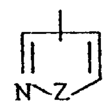 ; 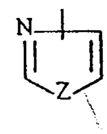 ;

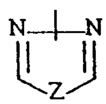 ; 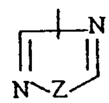 ; 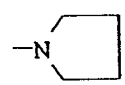 ; 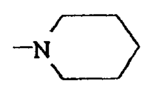 ;

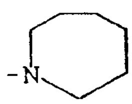 ; 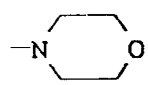 oder 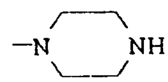

stehen,

wobei Z jeweils für Sauerstoff oder Schwefel steht,

außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, oder

$R^3$ und $R^4$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

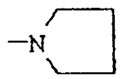 ; 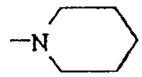 ; 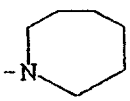 ; 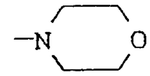 ;

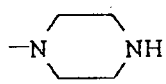 ; 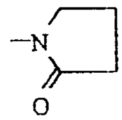 ; 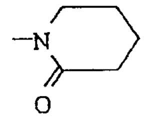 ; 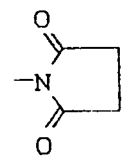 ;

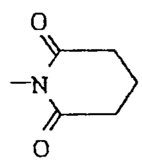 ; 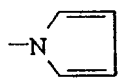 oder 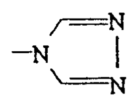

stehen,

X für Sauerstoff oder Schwefel steht und

Y für Sauerstoff oder Schwefel steht, wobei

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder

$R^5$ und $R^6$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

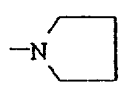 ; 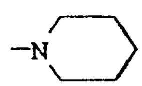 ; 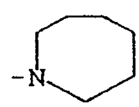 ; 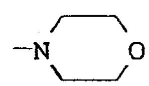 ;

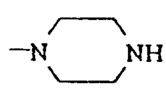 ; 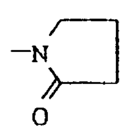 ; 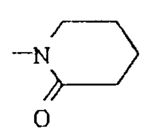 ; 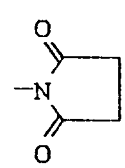 ;

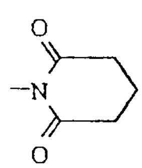 ; 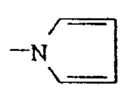 oder 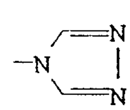

stehen,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und

n für eine Zahl 0, 1 oder 2 steht,

wobei jedoch $R^2$ nur dann für einen Rest $-S(O)_n-R^7$ steht, wenn $R^3$ und $R^4$ nicht gleichzeitig für Methyl stehen, ausgenommen die Verbindungen 5-Benzylthio-3-hydroxy-2(H)-phenylcarbamoyl-1,2,4-triazol, 2(H)-Carbamoyl-3-hydroxy-5-methylthio-1,2,4-triazol und 5-Benzylthio-2(H)-carbamoyl-3-hydroxy-1,2,4-triazol.

**2.** Verfahren zur Herstellung von substituierten Triazolinonen der allgemeinen Formel (I),

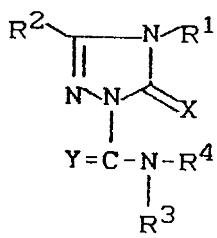

(I)

in welcher

$R^1$ für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,

$R^2$ für einen Rest

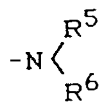

oder für einen Rest $S(O)_n-R^7$ steht,

$R^3$ und $R^4$ unabhängig voneinander jeweils für Wasserstoff, Methyl, Ethyl, n- oder i-Propyl, n-, i-, s-oder t-Butyl, n- oder i-Pentyl, n - oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden durch Fluor,Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen; außerdem für im Heterocyclylteil gegebenenfalls ein- bis dreifach, gleich oder verschieden durch Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl mit den Heterocyclen

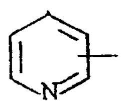 ; 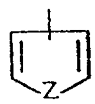 ; 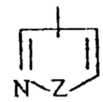 ; 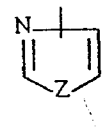 ;

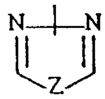 ; 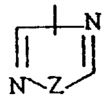 ; 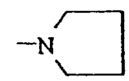 ; 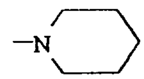 ;

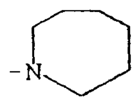 ; 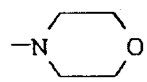 oder 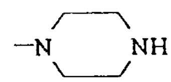

stehen,

wobei Z jeweils für Sauerstoff oder Schwefel steht,

außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, oder

$R^3$ und $R^4$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

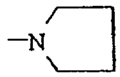 ; 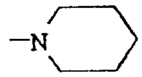 ; 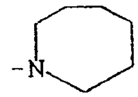 ; 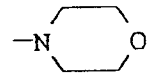 ;

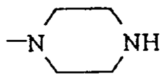 ; 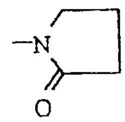 ; 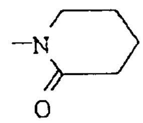 ; 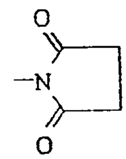 ;

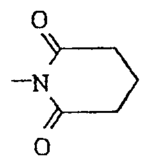 ; 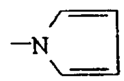 oder 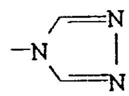

stehen,

X für Sauerstoff oder Schwefel steht und

Y für Sauerstoff oder Schwefel steht, wobei

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder

$R^5$ und $R^6$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

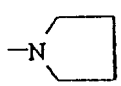 ; 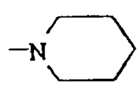 ; 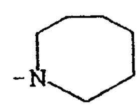 ; 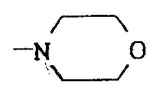 ;

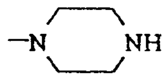 ; 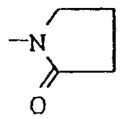 ; 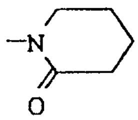 ; 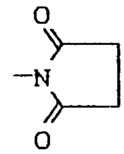 ;

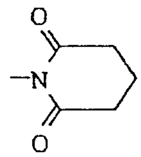 ; 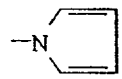 oder 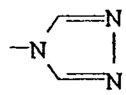

stehen,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und

n für eine Zahl 0, 1 oder 2 steht,

wobei jedoch $R^2$ nur dann für einen Rest $-S(O)_n-R^7$ steht, wenn $R^3$ und $R^4$ nicht gleichzeitig für Methyl stehen

stehen, ausgenommen die Verbindungen 5-Benzylthio-3-hydroxy-2(H)-phenylcarbamoyl-1,2,4-triazol, 2(H)-Carbamoyl-3-hydroxy-5-methylthio-1,2,4-triazol und 5-Benzylthio-2(H)-carbamoyl-3-hydroxy-1,2,4-triazol,

dadurch gekennzeichnet, daß man

a) 1-Chlor-(thio)-carbonyltriazolinone der allgemeinen Formel (II),

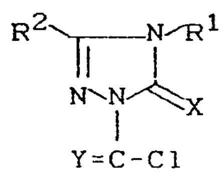 (II)

in welcher

$R^1$, $R^2$, X und Y die oben angegebene Bedeutung haben,

mit Aminen der allgemeinen Formel (III),

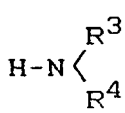 (III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt; oder

b) für den Fall, daß $R^3$ Wasserstoff bedeutet, in 1-Stellung unsubstituierte Triazolinone der allgemeinen Formel (IV),

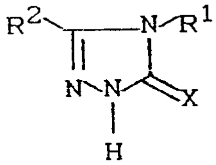

(IV)

in welcher

$R^1$, $R^2$ und X die oben angegebene Bedeutung haben,

mit Iso(thio)cyanaten der Formel (V),

$R^4$-N=C=Y (V)

in welcher

$R^4$ und Y die oben angegebene Bedeutung haben,

gegebenenfalls in Gegenwart eines Verdünnungsmittels und gegebenenfalls in Gegenwart eines Reaktionshilfsmittels umsetzt.

**3.** Herbizide Mittel, gekennzeichnet durch einen Gehalt an mindestens einem substituierten Triazolinon der allgemeinen Formel (I) gemäß Anspruch 1.

**4.** Verfahren zur Bekämpfung von Unkräutern, dadurch gekennzeichnet, daß man substituierte Triazolinone der allgemeinen Formel (I) gemäß Anspruch 1 auf die Unkräuter und/oder ihren Lebensraum einwirken läßt.

**5.** Verwendung von substituierten Triazolinonen der allgemeinen Formel (I) gemäß Anspruch 1 zur Bekämpfung von Unkräutern.

**6.** Verfahren zur Herstellung von herbiziden Mitteln, dadurch gekennzeichnet, daß man substituierte Triazolinone der allgemeinen Formel (I) gemäß Anspruch 1 mit Streckmitteln und/oder oberflächenaktiven Substanzen vermischt.

**7.** Verbindungen der allgemeinen Formel (II),

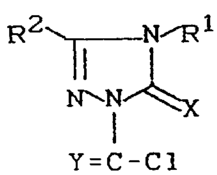

(II)

in welcher

$R^1$ für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,

$R^2$ für einen Rest

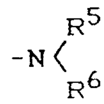

oder für einen Rest $S(O)_n$-$R^7$ steht,

X für Sauerstoff oder Schwefel steht und

Y für Sauerstoff oder Schwefel steht,

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder

$R^5$ und $R^6$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

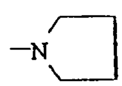 ; 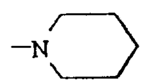 ; 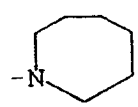 ; 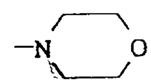 ;

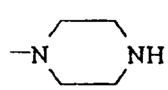 ; 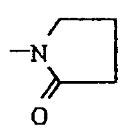 ; 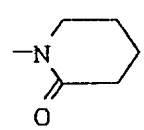 ; 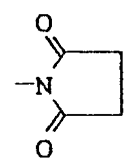 ;

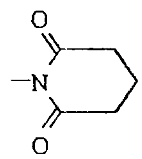 ; 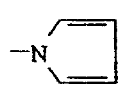 oder 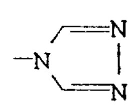

stehen,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und

n für eine Zahl 0, 1 oder 2 steht.

**8.** Verfahren zur Herstellung von Verbindungen der allgemeinen Formel (II),

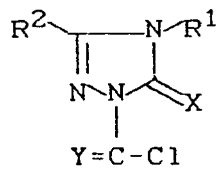

(II)

in welcher

$R^1$ für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,

$R^2$ für einen Rest

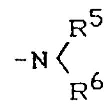

oder für einen Rest $S(O)_n$-$R^7$ steht,

X für Sauerstoff oder Schwefel steht und

Y für Sauerstoff oder Schwefel steht, wobei

$R^5$ und $R^6$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder

$R^5$ und $R^6$ gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel

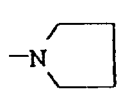 ; 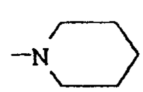 ; 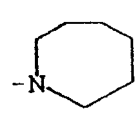 ; 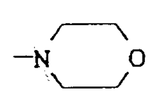 ;

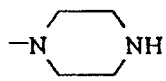 ; 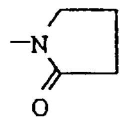 ; 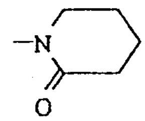 ; 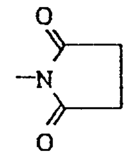 ;

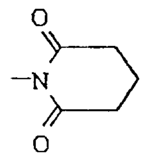 ; 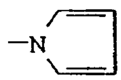 oder 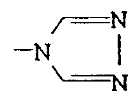

stehen,

$R^7$ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und

n für eine Zahl 0, 1 oder 2 steht,

dadurch gekennzeichnet, daß man

a) in 1-Stellung unsubstituierte Triazolinone der allgemeinen Formel (IV),

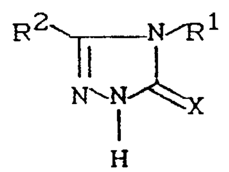 (IV)

in welcher

$R^1$, $R^2$ und X die oben angegebene Bedeutung haben,

mit (Thio)Phosgen der allgemeinen Formel (VI),

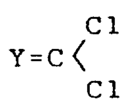 (VI)

in welcher

Y die oben angegebene Bedeutung hat,

gegebenenfalls in Gegenwart eines Verdünnungsmittels und gegebenenfalls in Gegenwart eines Säurebindemittels umsetzt,

oder daß man

b) Aminoguanidiniumhydrochloride der allgemeinen Formel (VII),

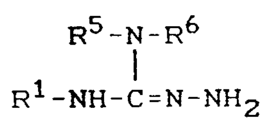 x HCl (VII)

in welcher

$R^1$, $R^5$ und $R^6$ die oben angegebene Bedeutung haben,

mit doppelt-molarem Überschuß an (Thio)Phosgen der Formel (VI),

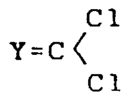 (VI)

in welcher

Y die oben angegebene Bedeutung hat,

gegebenenfalls in Gegenwart eines Verdünnungsmittels und gegebenenfalls in Gegenwart eines Säurebindemittels umsetzt.

## Claims

**1.** Substituted triazolinones of the general formula (1)

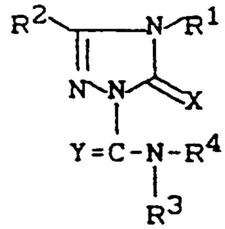 (I)

in which

$R^1$ represents methyl, ethyl, n- or i-propyl, n-, i-, s- or t-butyl, n- or i-pentyl, n- or i-hexyl, allyl, propargyl, methoxy, ethoxy or methoxymethyl, or represents straight-chain or branched halogenoalkyl with 1 to 4 carbon atoms and 1 to 9 identical or different halogen atoms, in particular fluorine, chlorine or bromine, or represents cyclopentyl, cyclohexyl, cyclopropyl, clyclopropylmethyl, cyclohexylmethyl or cyclohexylethyl, or represents phenyl or benzyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

$R^2$ represents a radical

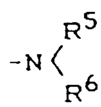,

or represents a radical $-S(O)_n-R^7$,

$R^3$ and $R^4$ independently of one another each represent hydrogen, methyl, ethyl, n- or i-propyl, n-, i-, s- or t-butyl, n- or i-pentyl, n- or i-hexyl, n- or i-heptyl, n- or i-octyl, n- or i-nonyl, n- or i-decyl, n- or i-dodecyl, allyl, n- or i-butenyl, n- or i-pentenyl, n- or i-hexenyl, propargyl, n- or i-butinyl, n- or i-pentinyl, n- or i-hexinyl, or represent straight-chain or branched halogenoalkyl with 1 to 6 carbon atoms and 1 to 9 identical or different halogen atoms, in particular fluorine, chlorine or bromine, or represent in each case straight-chain or branched halogenoalkenyl or halogenoalkinyl with in each case 3 to 5 carbon atoms and 1 to 3 halogen atoms, in particular fluorine or chlorine, or represent in each case straight-chain or branched cyanoalkyl with 1 to 6 carbon atoms in the alkyl part, hydroxyalkyl with 1 to 6 carbon atoms and 1 to 3 hydroxyl groups, alkoxyalkyl, alkoxycarbonylalkyl or alkoxycarbonylalkenyl, or alkylaminoalkyl or dialkylaminoalkyl with in each case up to 4 carbon atoms in the individual alkyl and alkenyl parts, or represent cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, in each case optionally substituted by one to three identical or different substituents from the group comprising fluorine, chlorine, bromine, methyl,ethyl, n- or i-propyl, n-, i-, s- or t-butyl, cyano, methanediyl, ethanediyl, butanediyl and butadienediyl; or furthermore represent heterocyclylmethyl, heterocyclylpropyl or heterocyclylethyl with the heterocyclic radicals

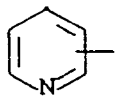 ; 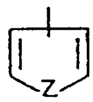 ; 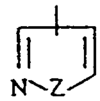 ; 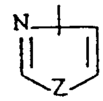 ;

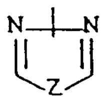 ; 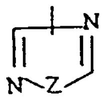 ; 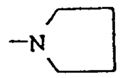 ; 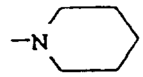 ;

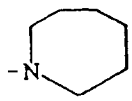 ; 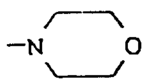 or 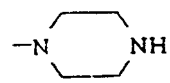

wherein

Z in each case represents oxygen or sulphur, optionally substituted in the heterocyclyl part by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

or furthermore represents in each case straight-chain or branched alkoxy with 1 to 6 carbon atoms, alkenyloxy with 3 to 6 carbon atoms or alkinyloxy with 3 to 6 carbon atoms, or represents optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethyloxy, phenoxy, benzoyl, phenyl or naphthyl, in each case optionally substituted in the phenyl part by one to three identical or different substituents from the group comprising fluorine, chlorine, bromine, hydroxy, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, or

$R^3$ and $R^4$, together with the nitrogen atom to which they are bonded, represent a heterocyclic radical of the formula

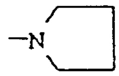 ; 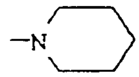 ; 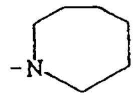 ; 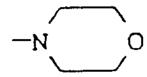 ;

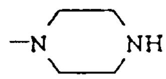 ; 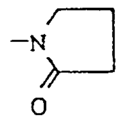 ; 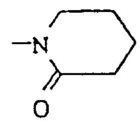 ; 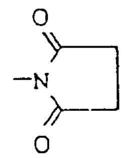 ;

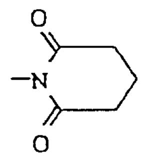 ; 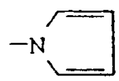 or 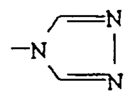

optionally substituted by one to three identical or different substituents from the group comprising methyl, ethyl, n- or i-propyl, chlorine and trifluoromethyl,

X represents oxygen or sulphur and

Y represents oxygen or sulphur, wherein

$R^5$ and $R^6$ independently of one another each represent methyl, ethyl, n- or i-propyl, n-, i-, s- or t-butyl, n- or i-pentyl, allyl or propargyl, or represent in each case straight-chain or branched halogenoalkyl with 1 to 4 carbon atoms, halogenoalkenyl with 3 to 6 carbon atoms or halogenoalkinyl with 3 to 6 carbon atoms and in each case 1 to 9 identical or different halogen atoms, or represent methoxymethyl, methoxyethyl, methoxy or ethoxy, or represent cyclopropyl, cyclopropylmethyl, cyclopentyl, cyclohexyl, cyclohexylmethyl, cyclohexylethyl or cyclopentylmethyl, or represent benzyl, phenylethyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio, or

$R^5$ and $R^6$ , together with the nitrogen atom to which they are bonded, represent a heterocyclic radical of the formula

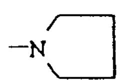 ; 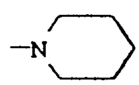 ; 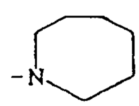 ; 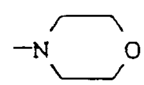 ;

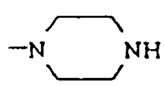 ; 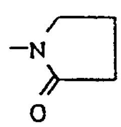 ; 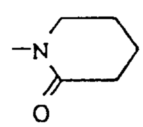 ; 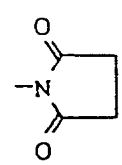 ;

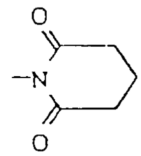 ; 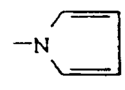 or 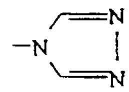

optionally substituted by one to three identical or different substituents from the group comprising methyl, ethyl, n- or i-propyl, chlorine and trifluoromethyl,

$R^7$ represents methyl, ethyl, n- or i-propyl, n-, i-, s- or t-butyl, allyl, propargyl, cyclopentyl, cyclohexyl, cyclohexylmethyl or cyclohexylethyl, or represents benzyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy and trifluoromethyl, and

n represents a number 0, 1 or 2,

but wherein $R^2$ only represents a radical $-S(O)_n-R^7$ if $R^3$ and $R^4$ do not simultaneously represent methyl, with the exception of the compounds 5-benzylthio-3-hydroxy-2(H)-phenylcarbamoyl-1,2,4-triazole,2(H)-carbamoyl-3-hydroxy-5-methylthio-1,2,4-triazole and 5-benzylthio-2(H)-carbamoyl-3-hydroxy-1,2,4-triazole.

**2.** Process for the preparation of substituted triazolinones of the general formula (I)

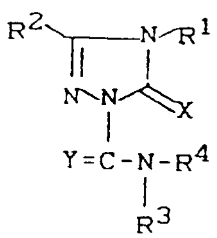 (I)

in which

$R^1$ represents methyl, ethyl, n- or i-propyl, n-, i-, s- or t-butyl, n- or i-pentyl, n- or i-hexyl, allyl, propargyl, methoxy, ethoxy or methoxymethyl, or represents straight-chain or branched halogenoalkyl with 1 to 4 carbon atoms and 1 to 9 identical or different halogen atoms, in particular fluorine, chlorine or bromine, or represents cyclopentyl, cyclohexyl, cyclopropyl, cyclopropylmethyl, cyclohexylmethyl or cyclohexylethyl, or represents phenyl or benzyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

$R^2$ represents a radical

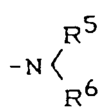 ,

or represents a radical $-S(O)_n-R^7$,

$R^3$ and $R^4$ independently of one another each represent hydrogen, methyl, ethyl, n- or i-propyl, n-, i-, s- or t-butyl, n- or i-pentyl, n- or i-hexyl, n- or i-heptyl, n- or i-octyl, n- or i-nonyl, n- or i-decyl, n- or i-dodecyl, allyl, n- or i-butenyl, n- or i-pentenyl, n- or i-hexenyl, propargyl, n- or i-butinyl, n- or i-pentinyl, n- or i-hexinyl, or represent straight-chain or branched halogenoalkyl with 1 to 6 carbon atoms and 1 to 9 identical or different halogen atoms, in particular fluorine, chlorine or bromine, or represent in each case straight-chain or branched halogenoalkenyl or halogenoalkinyl

with in each case 3 to 5 carbon atoms and 1 to 3 halogen atoms, in particular fluorine or chlorine, or represent in each case straight-chain or branched cyanoalkyl with 1 to 6 carbon atoms in the alkyl part, hydroxyalkyl with 1 to 6 carbon atoms and 1 to 3 hydroxyl groups, alkoxyalkyl, alkoxycarbonylalkyl or alkoxycarbonylalkenyl, or alkylaminoalkyl or dialkylaminoalkyl with in each case up to 4 carbon atoms in the individual alkyl and alkenyl parts, or represent cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, in each case optionally substituted by one to three identical or different substituents from the group comprising fluorine, chlorine, bromine, methyl, ethyl, n- or i-propyl, n-, i-, s- or t-butyl, cyano, methanediyl, ethanediyl, butanediyl and butadienediyl; or furthermore represent heterocyclylmethyl, heterocyclylpropyl or heterocyclylethyl with the heterocyclic radicals

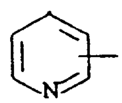 ; 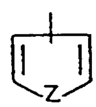 ; 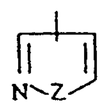 ; 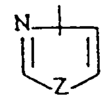 ;

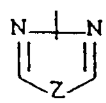 ; 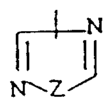 ; 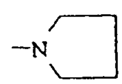 ; 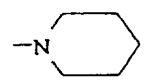 ;

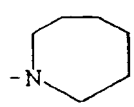 ; 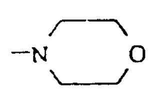 or 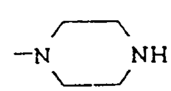

wherein

Z in each case represents oxygen or sulphur, optionally substituted in the heterocyclyl part by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

or furthermore represents in each case straight-chain or branched alkoxy with 1 to 6 carbon atoms, alkenyloxy with 3 to 6 carbon atoms or alkinyloxy with 3 to 6 carbon atoms, or represents optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethyloxy, phenoxy, benzoyl, phenyl or naphthyl, in each case optionally substituted in the phenyl part by one to three identical or different substituents from the group comprising fluorine, chlorine, bromine, hydroxy, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, or

$R^3$ and $R^4$, together with the nitrogen atom to which they are bonded, represent a heterocyclic radical of the formula

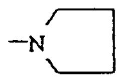 ; 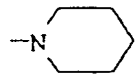 ; 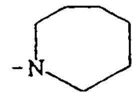 ; 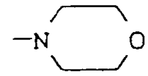 ;

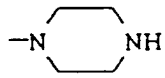 ; 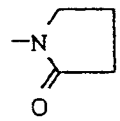 ; 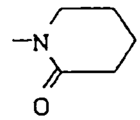 ; 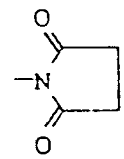 ;

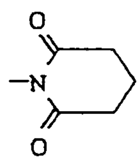 ; 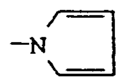 or 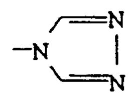

optionally substituted by one to three identical or different substituents from the group comprising methyl, ethyl, n- or i-propyl, chlorine and trifluoromethyl,

X represents oxygen or sulphur and

Y represents oxygen or sulphur, wherein

$R^5$ and $R^6$ independently of one another each represent methyl, ethyl, n- or i-propyl, n-, i-, s- or t-butyl, n- or i-pentyl, allyl or propargyl, or represent in each case straight-chain or branched halogenoalkyl with 1 to 4 carbon atoms, halogenoalkenyl with 3 to 6 carbon atoms or halogenoalkinyl with 3 to 6 carbon atoms and in each case 1 to 9 identical or different halogen atoms, or represent methoxymethyl, methoxyethyl, methoxy or ethoxy, or represent cyclopropyl, cyclopropylmethyl, cyclopentyl, cyclohexyl, cyclohexylmethyl, cyclohexylethyl or cyclopentylmethyl, or represent benzyl, phenylethyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio, or

$R^5$ and $R^6$, together with the nitrogen atom to which they are bonded, represent a heterocyclic radical of the formula

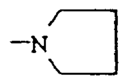 ; 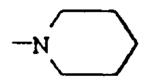 ; 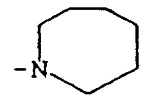 ; 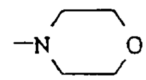 ;

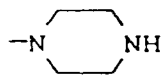 ; 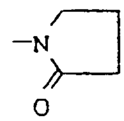 ; 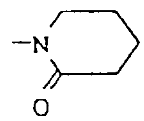 ; 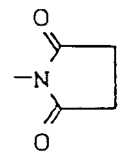 ;

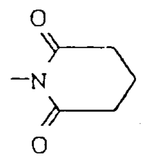 ; 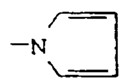 or 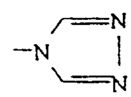

optionally substituted by one to three identical or different substituents from the group comprising methyl, ethyl, n- or i-propyl, chlorine and trifluoromethyl,

$R^7$ represents methyl, ethyl, n- or i-propyl, n-, i-, s- or t-butyl, allyl, propargyl, cyclopentyl, cyclohexyl, cyclohexylmethyl or cyclohexylethyl, or represents benzyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy and trifluoromethyl, and

n represents a number 0, 1 or 2,

but wherein $R^2$ only represents a radical $-S(O)_n-R^7$ if $R^3$ and $R^4$ do not simultaneously represent methyl, with the exception of the compounds 5-benzylthio-3-hydroxy-2(H)-phenylcarbamoyl-1,2,4-triazole,2(H)-carbamoyl-3-hydroxy-5-methylthio-1,2,4-triazole and 5-benzylthio-2(H)-carbamoyl-3-hydroxy-1,2,4-triazole,

characterized in that

a) 1-chloro-(thio)-carbonyltriazolinones of the general formula (II)

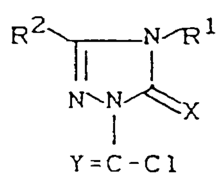 (II)

in which

$R^1$, $R^2$, X and Y have the abovementioned meaning, are reacted with amines of the formula (III)

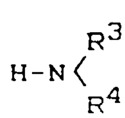 (III)

in which

$R^3$ and $R^4$ have the abovementioned meaning, if appropriate in the presence of a diluent and if appropriate in the presence of an acid-binding agent; or

b) in the case where $R^3$ denotes hydrogen, triazolinones unsubstituted in the 1-position, of the general formula (IV)

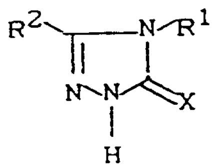

(IV)

in which

$R^1$, $R^2$ and X have the abovementioned meaning, are reacted with iso(thio)cyanates of the formula (V)

$R^4$-N=C=Y (V)

in which

$R^4$ and Y have the abovementioned meaning, if appropriate in the presence of a diluent and if appropriate in the presence of a reaction auxiliary.

**3.** Herbicidal agents, characterized in that they contain at least one substituted triazolinone of the general formula (I) according to Claim 1.

**4.** Method of combating weeds, characterized in that substituted triazolinones of the general formula (I) according to Claim 1 are allowed to act on the weeds and/or their environment.

**5.** Use of substituted triazolinones of the general formula (I) according to Claim 1 for combating weeds.

**6.** Process for the preparation of herbicidal agents, characterized in that substituted triazolinones of the general formula (I) according to Claim 1 are mixed with extenders and/or surface-active substances.

**7.** Compounds of the general formula (II)

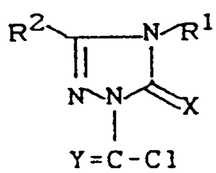

(II)

in which

$R^1$ represents methyl, ethyl, n- or i-propyl, n-, i-, s- or t-butyl, n- or i-pentyl, n- or i-hexyl, allyl, propargyl, methoxy, ethoxy or methoxymethyl, or represents straight-chain or branched halogenoalkyl with 1 to 4 carbon atoms and 1 to 9 identical or different halogen atoms, in particular fluorine, chlorine or bromine, or represents cyclopentyl, cyclohexyl, cyclopropyl, cyclopropylmethyl, cyclohexylmethyl or cyclohexylethyl, or represents phenyl or benzyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

$R^2$ represents a radical

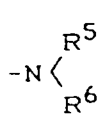,

or represents a radical $-S(O)_n-R^7$,

X represents oxygen or sulphur and

Y represents oxygen or sulphur, wherein

$R^5$ and $R^6$ independently of one another each represent methyl, ethyl, n- or i-propyl, n-, i-, s- or t-butyl, n- or i-pentyl, allyl or propargyl, or represent in each case straight-chain or branched halogenoalkyl with 1 to 4 carbon atoms, halogenoalkenyl with 3 to 6 carbon atoms or halogenoalkinyl with 3 to 6 carbon atoms and in each case 1 to 9 identical or different halogen atoms, or represent methoxymethyl, methoxyethyl, methoxy or ethoxy, or represent cyclopropyl, cyclopropylmethyl, cyclopentyl, cyclohexyl, cyclohexylmethyl, cyclohexylethyl or cyclopentylmethyl, or represent benzyl, phenylethyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio, or

$R^5$ and $R^6$, together with the nitrogen atom to which they are bonded, represent a heterocyclic radical of the formula

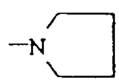 ; 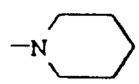 ; 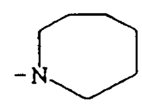 ; 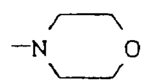 ;

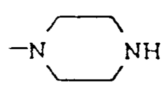 ; 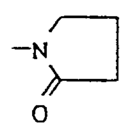 ; 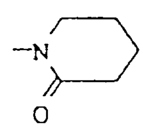 ; 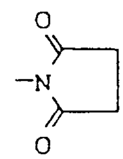 ;

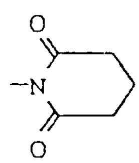 ; 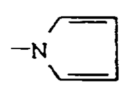 or 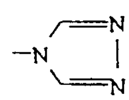

optionally substituted by one to three identical or different substituents from the group comprising methyl, ethyl, n- or i-propyl, chlorine and trifluoromethyl,

$R^7$ represents methyl, ethyl, n- or i-propyl, n-, i-, s- or t-butyl, allyl, propargyl, cyclopentyl, cyclohexyl, cyclohexylmethyl or cyclohexylethyl, or represents benzyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy and trifluoromethyl, and

n represents a number 0, 1 or 2.

**8.** Process for the preparation of compounds of the general formula (II)

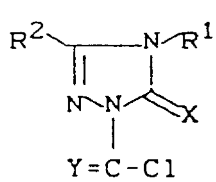 (II)

in which

$R^1$ represents methyl, ethyl, n- or i-propyl, n-, i-, s- or t-butyl, n- or i-pentyl, n- or i-hexyl, allyl, propargyl, methoxy, ethoxy or methoxymethyl, or represents straight-chain or branched halogenoalkyl with 1 to 4 carbon atoms and 1 to 9 identical or different halogen atoms, in particular fluorine, chlorine or bromine, or represents cyclopentyl, cyclohexyl, cyclopropyl, cyclopropylmethyl, cyclohexylmethyl or cyclohexylethyl, or represents phenyl or benzyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio,

$R^2$ represents a radical

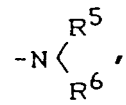

or represents a radical $-S(O)_n-R^7$,

X represents oxygen or sulphur and

Y represents oxygen or sulphur, wherein

$R^5$ and $R^6$ independently of one another each represent methyl, ethyl, n- or i-propyl, n-, i-, s- or t-butyl, n- or i-pentyl, allyl or propargyl, or represent in each case straight-chain or branched halogenoalkyl with 1 to 4 carbon atoms, halogenoalkenyl with 3 to 6 carbon atoms or halogenoalkinyl with 3 to 6 carbon atoms and in each case 1 to 9 identical or different halogen atoms, or represent methoxymethyl, methoxyethyl, methoxy or ethoxy, or represent cyclopropyl, cyclopropylmethyl, cyclopentyl, cyclohexyl, cyclohexylmethyl, cyclohexylethyl or cyclopentylmethyl, or represent benzyl, phenylethyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy and trifluoromethylthio, or

$R^5$ and $R^6$, together with the nitrogen atom to which they are bonded, represent a heterocyclic radical of the formula

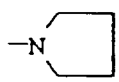 ; 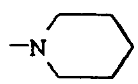 ; 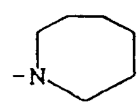 ; 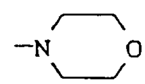 ;

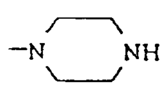 ; 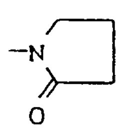 ; 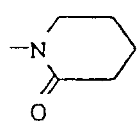 ; 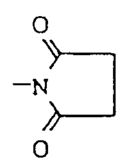 ;

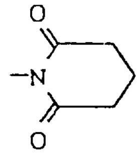 ; 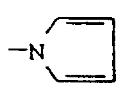 or 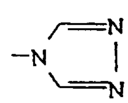

optionally substituted by one to three identical or different substituents from the group comprising methyl, ethyl, n- or i-propyl, chlorine and trifluoromethyl,

$R^7$ represents methyl, ethyl, n- or i-propyl, n-, i-, s- or t-butyl, allyl, propargyl, cyclopentyl, cyclohexyl, cyclohexylmethyl or cyclohexylethyl, or represents benzyl or phenyl, in each case optionally substituted by one to three identical or different substituents from the group comprising fluorine, chlorine, bromine, cyano, nitro, methyl, ethyl, n- or i-propyl, n-, i-, s- or t-butyl, methoxy, ethoxy and trifluoromethyl, and

n represents number 0, 1 or 2,

characterized in that

a) triazolinones unsubstituted in the 1-position, of the general formula (IV)

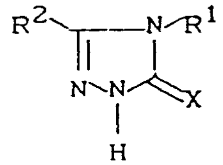

(IV)

in which

$R^1$, $R^2$ and X have the abovementioned meaning, are reacted with (thio)phosgene of the general formula (VI)

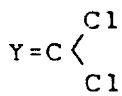

(VI)

in which

Y has the abovementioned meaning,

if appropriate in the presence of a diluent and if appropriate in the presence of an acid-binding agent,

or in that

b) aminoguanidinium hydrochlorides of the general formula (VII)

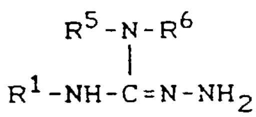

x HCl (VII)

in which

$R^1$, $R^5$ and $R^6$ have the abovementioned meaning,

are reacted with twice the molar excess of (thio)phosgene of the formula (VI)

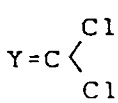

(VI)

in which

Y has the abovementioned meaning,

if appropriate in the presence of a diluent and if appropriate in the presence of an acid-binding agent.

## Revendications

**1.** Triazolinones substituées de formule générale I

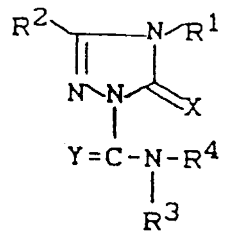

(I)

dans laquelle $R^1$ représente un groupe méthyle, éthyle, n- ou iso-propyle, n-, iso, sec-, ou tert-butyle, n- ou iso-pentyle, n- ou iso-hexyle, allyle, propargyle, méthoxy, éthoxy, méthoxyméthyle, un groupe halogénoalkyle à chaîne droite ou ramifiée contenant 1 à 4 atomes de carbone et 1 à 9 atomes d'halogènes identiques ou différents, en particulier de chlore, de fluor ou de brome, un groupe cyclopentyle, cyclohexyle, cyclopropyle, cyclopropylméthyle, cyclohexyméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio,

$R^2$ représente un groupe

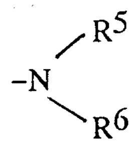

ou un groupe $S(O)_n$-$R^7$

$R^3$ et $R^4$ représentent chacun, indépendamment l'un de l'autre, l'hydrogène, un groupe méthyle, éthyle, n- ou iso-propyle, n-, iso-, sec- ou tert-butyle, n- ou iso-pentyle, n- ou iso-hexyle, n- ou iso-heptyle, n- ou iso-octyle, n- ou iso-nonyle, n- ou iso-décyle, n- ou iso-dodécyle, un groupe allyle, n- ou iso-buténуle, n- ou iso-penténуle, n- ou iso-hexényle, propargyle, n- ou iso-buténуle, n- ou isopentynyle, n- ou iso-hexynyle, un groupe halogénoalkyle à chaîne droite ou ramifiée contenant 1 à 6 atomes de carbone et 1 à 9 atomes d'halogènes identiques ou différents, en particulier de fluor, de chlore ou de brome, un groupe halogénoalcényle ou halogénoalcynyle, chacun à chaîne droite ou ramifiée et contenant chacun 3 à 5 atomes de carbone et 1 à 3 atomes d'halogènes, en particulier de fluor ou de chlore, un groupe cyanalkyle contenant 1 à 6 atomes de carbone dans la partie alkyle, hydroxyalkyle contenant 1 à 6 atomes de carbone et 1 à 3 groupes hydroxy, alcoxyalkyle, alcoxycarbonylalkyle ou alcoxycarbonylalcényle, chacun d'eux à chaîne droite ou ramifiée, un groupe alkylaminoalkyle ou diakylaminoalkyle contenant chacun jusqu'à 4 atomes de carbone dans chacune des parties alkyle et alcényle, ou un groupe cyclopropyle, cyclopropylméthyle, cyclopropyléthyle, cyclopentyle, cyclohexyle, cycloheptyle, cyclooctyle, cyclohexylméthyle, cyclohexyléthyle, cyclohexényle ou cyclohexénylméthyle portant chacun le cas échéant 1 à 3 substituants identiques ou différents choisis parmi le fluor, le chlore, le brome, les groupes méthyle, éthyle, n- ou iso-propyle, n- ou iso-, sec- ou tert-butyle, cyano, méthane-diyle, éthane-diyle, butane-diyle ou butadène-diyle ; en outre, un groupe hétérocyclylméthyle, hétérocyclylpropyle ou hétérocyclyléthyle portant éventuellement dans la partie hétérocyclyle 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio, et dont les hétérocycles sont :

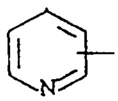 ; 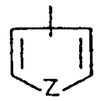 ; 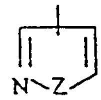 ; 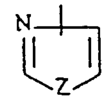 ;

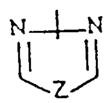 ; 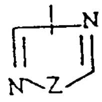 ; 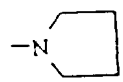 ; 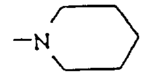 ;

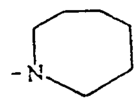 ; 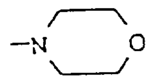 ou 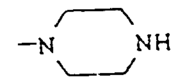

dans lesquels Z à chaque fois représente l'oxygène ou le soufre, en encore un groupe alcoxy en $C_1$-$C_6$, alcényloxy en $C_3$-$C_6$ ou alcynyloxy en $C_3$-$C_6$, chacun d'eux à chaîne droite ou ramifiée, ou un groupe benzyle, phényléthyle, phénylpropyle, phénybutyle, phénylpentyle, phénylhexyle, phénylheptyle, phénylcyanaméthyle, phénylcyanéthyle, phénylcyanopropyle, benzyloxy, phényléthyloxy, phénoxy, benzoyle, phényle ou naphtyle, chacun d'eux portant éventuellement dans la partie phényle 1 à 3 substituants identiques ou différents choisis parmi le fluor, le chlore, le brome, les groupes hydroxy, cyano, nitro, méthyle, éthyle, n- ou iso-propyle, n-, iso-, sec- ou tert-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy, ou bien

$R^3$ et $R^4$ forment ensemble et avec l'atome d'azote auquel ils sont reliés un hétérocycle de formule

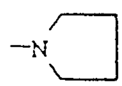 ; 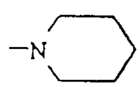 ; 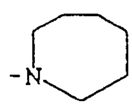 ; 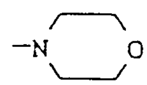 ;

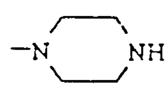 ; 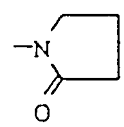 ; 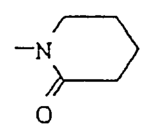 ; 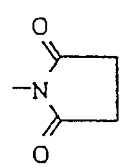 ;

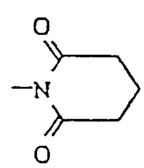 ; 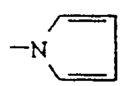 ou 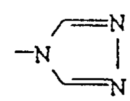

portant éventuellement 1 à 3 substituants identiques ou différents choisis parmi les groupes méthyle, éthyle, n- ou iso-propyle, le chlore ou les groupes trifluorométhyle,

X représente l'oxygène ou le soufre, et

Y représente l'oxygène ou le soufre,

$R^5$ et $R^6$ représentent chacun, indépendamment l'un de l'autre, un groupe méthyle, éthyle, n- ou iso-propyle, n-, iso-, sec- ou tert-butyle, n- ou iso-pentyle, allyle, propargyle, un groupe halogénoalkyle en $C_1$-$C_4$, halogénoalcényle en $C_3$-$C_6$, ou halogénoalcynyle en $C_3$-$C_6$, chacun d'eux à chaîne droite ou ramifiée et contenant chacun 1 à 9 atomes d'halogènes identiques ou différents, un groupe méthoxyméthyle, méthoxyéthyle, méthoxy, éthoxy, un groupe cyclopropyle, cyclopropylméthyle, cyclopentyle, cyclohexyle, cyclohexylméthyle, cyclohexyléthyle, cyclopentylméthyle ou un groupe benzyle, phényléthyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle,

n-, iso-, sec- ou tertbutyle, méthoxy, éthoxy, méthylthio, trifluorométhyle, trifluorométhoxy ou trifluorométhylthio, ou bien
$R^5$ et $R^6$ forment ensemble et avec l'atome d'azote auquel ils sont reliés un hétérocycle substitué de formule

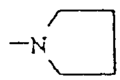 ; 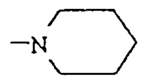 ; 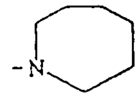 ; 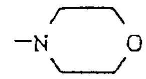 ;

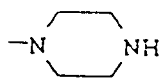 ; 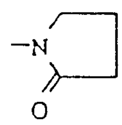 ; 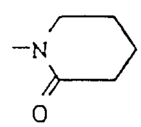 ; 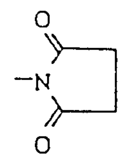 ;

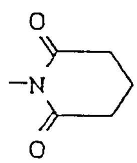 ; 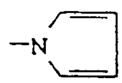 ou 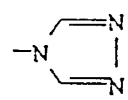

portant éventuellement 1 à 3 substituants identiques ou différents choisis parmi les groupes méthyle, éthyle, n- ou iso-propyle, le chlore ou les groupes trifluorométhyle,
$R^7$ représente un groupe méthyle, éthyle, n- ou iso-propyle, n-, iso-, sec- ou tert-butyle, allyle, propargyle, cyclopentyle, cyclohexyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy ou trifluorométhyle, et
n est égal à 0, 1 ou 2,
étant spécifié toutefois que $R^2$ ne peut représenter un groupe $-S(O)_n-R^7$ que si $R^3$ et $R^4$, simultanément, ne représentent pas des groupes méthyle, et à l'exception des composés : 5-benzylthio-3-hydroxy-2(H)-phénylcarbamoyl-1,2,4-triazole,
2(H)-carbamoyl-3-hydroxy-5-méthylthio-1,2,4-triazole et 5-benzylthio-2(H)-carbamoyl-3-hydroxy-1,2,4 triazole.

**2.** Procédé de préparation des triazolinones substituées de formule générale I

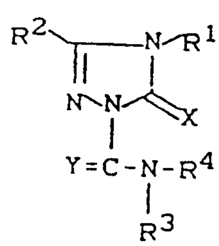

(I)

dans laquelle $R^1$ représente un groupe méthyle, éthyle, n- ou iso-propyle, n-, iso, sec-, ou tert-butyle, n- ou iso-pentyle, n- ou iso-hexyle, allyle, propargyle, méthoxy, éthoxy, méthoxyméthyle, un groupe halogénoalkyle à chaîne droite ou ramifiée contenant 1 à 4 atomes de carbone et 1 à 9 atomes d'halogènes identiques ou différents, en particulier de chlore, de fluor ou de brome, un groupe cyclopentyle, cyclohexyle, cyclopropyle, cyclopropylméthyle, cyclohexyméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio,
$R^2$ représente un groupe

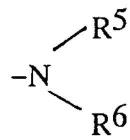

ou un groupe $S(O)_n-R^7$

$R^3$ et $R^4$ représentent chacun, indépendamment l'un de l'autre, l'hydrogène, un groupe méthyle, éthyle, n- ou iso-propyle, n-, iso-, sec- ou tert-butyle, n- ou iso-pentyle, n- ou iso-hexyle, n- ou iso-heptyle, n- ou iso-octyle, n- ou iso-nonyle, n- ou iso-décyle, n- ou iso-dodécyle, un groupe allyle, n- ou iso-butényle, n- ou iso-pentényle, n- ou iso-hexényle, propargyle, n- ou iso-butényle, n- ou isopentynyle, n- ou iso-hexynyle, un groupe halogénoalkyle à chaîne droite ou ramifiée contenant 1 à 6 atomes de carbone et 1 à 9 atomes d'halogènes identiques ou différents, en particulier de fluor, de chlore ou de brome, un groupe halogénoalcényle ou halogénoalcynyle, chacun à chaîne droite ou ramifiée et contenant chacun 3 à 5 atomes de carbone et 1 à 3 atomes d'halogènes, en particulier de fluor ou de chlore, un groupe cyanalkyle contenant 1 à 6 atomes de carbone dans la partie alkyle, hydroxyalkyle contenant 1 à 6 atomes de carbone et 1 à 3 groupes hydroxy, alcoxyalkyle, alcoxycarbonylalkyle ou alcoxycarbonylalcényle, chacun d'eux à chaîne droite ou ramifiée, un groupe alkylaminoalkyle ou diakylaminoalkyle contenant chacun jusqu'à 4 atomes de carbone dans chacune des parties alkyle et alcényle, ou un groupe cyclopropyle, cyclopropylméthyle, cyclopropyléthyle, cyclopentyle, cyclohexyle, cycloheptyle, cyclooctyle, cyclohexylméthyle, cyclohexyléthyle, cyclohexényle ou cyclohexénylméthyle portant chacun le cas échéant 1 à 3 substituants identiques ou différents choisis parmi le fluor, le chlore, le brome, les groupes méthyle, éthyle, n- ou iso-propyle, n- ou iso-, sec- ou tert-butyle, cyano, méthane-diyle, éthane-diyle, butane-diyle ou butadène-diyle ; en outre, un groupe hétérocyclylméthyle, hétérocyclylpropyle ou hétérocyclyléthyle portant éventuellement dans la partie hétérocyclyle 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio, et dont les hétérocycles sont :

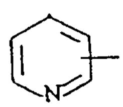 ; 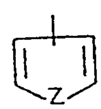 ; 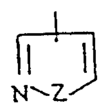 ; 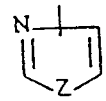 ;

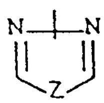 ; 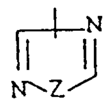 ; 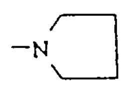 ; 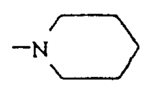 ;

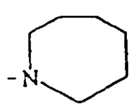 ; 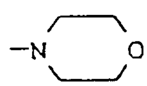 ou 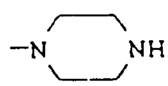

dans lesquels Z à chaque fois représente l'oxygène ou le soufre, en encore un groupe alcoxy en $C_1$-$C_6$, alcényloxy en $C_3$-$C_6$ ou alcynyloxy en $C_3$-$C_6$, chacun d'eux à chaîne droite ou ramifiée, ou un groupe benzyle, phényléthyle, phénylpropyle, phénybutyle, phénylpentyle, phénylhexyle, phénylheptyle, phénylcyanaméthyle, phénylcyanéthyle, phénylcyanopropyle, benzyloxy, phényléthyloxy, phénoxy, benzoyle, phényle ou naphtyle, chacun d'eux portant éventuellement dans la partie phényle 1 à 3 substituants identiques ou différents choisis parmi le fluor, le chlore, le brome, les groupes hydroxy, cyano, nitro, méthyle, éthyle, n- ou iso-propyle, n-, iso-, sec- ou tert-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy, ou bien

$R^3$ et $R^4$ forment ensemble et avec l'atome d'azote auquel ils sont reliés un hétérocycle de formule

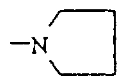 ; 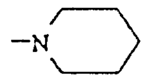 ; 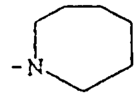 ; 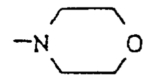 ;

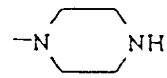 ; 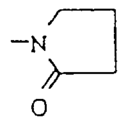 ; 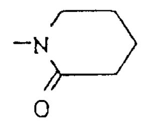 ; 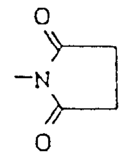 ;

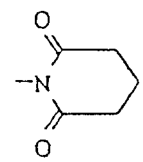 ; 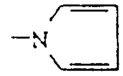 ou 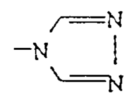

portant éventuellement 1 à 3 substituants identiques ou différents choisis parmi les groupes méthyle, éthyle, n- ou iso-propyle, le chlore ou les groupes trifluorométhyle,

X représente l'oxygène ou le soufre, et

Y représente l'oxygène ou le soufre,

$R^5$ et $R^6$ représentent chacun, indépendamment l'un de l'autre, un groupe méthyle, éthyle, n- ou iso-propyle, n-, iso-, sec- ou tert-butyle, n- ou iso-pentyle, allyle, propargyle, un groupe halogénoalkyle en $C_1$-$C_4$, halogénoalcényle en $C_3$-$C_6$, ou halogénoalcynyle en $C_3$-$C_6$, chacun d'eux à chaîne droite ou ramifiée et contenant chacun 1 à 9 atomes d'halogènes identiques ou différents, un groupe méthoxyméthyle, méthoxyéthyle, méthoxy, éthoxy, un groupe cyclopropyle, cyclopropylméthyle, cyclopentyle, cyclohexyle, cyclohexylméthyle, cyclohexyléthyle, cyclopentylméthyle ou un groupe benzyle, phényléthyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tertbutyle, méthoxy, éthoxy, méthylthio, trifluorométhyle, trifluorométhoxy ou trifluorométhylthio, ou bien

$R^5$ et $R^6$ forment ensemble et avec l'atome d'azote auquel ils sont reliés un hétérocycle substitué de formule

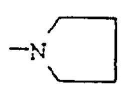 ; 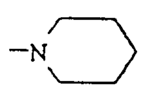 ; 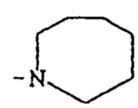 ; 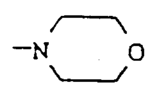 ;

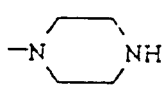 ; 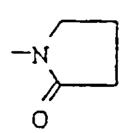 ; 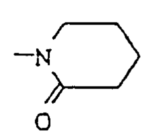 ; 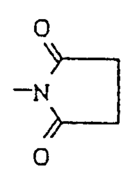 ;

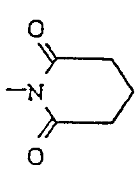 ; 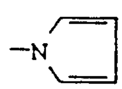 ou 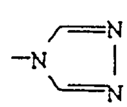 .

portant éventuellement 1 à 3 substituants identiques ou différents choisis parmi les groupes méthyle,

éthyle, n- ou iso-propyle, le chlore ou les groupes trifluorométhyle,
$R^7$ représente un groupe méthyle, éthyle, n- ou iso-propyle, n-, iso-, sec- ou tert-butyle, allyle, propargyle, cyclopentyle, cyclohexyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy ou trifluorométhyle, et
n est égal à 0, 1 ou 2,
étant spécifié toutefois que $R^2$ ne peut représenter un groupe $-S(O)_n-R^7$ que si $R^3$ et $R^4$, simultanément, ne représentent pas des groupes méthyle, et à l'exception des composés : 5-benzylthio-3-hydroxy-2(H)-phénylcarbamoyl-1,2,4-triazole, 2(H)-carbamoyl-3-hydroxy-5-méthylthio-1,2,4-triazole et 5-benzylthio-2(H)-carbamoyl-3-hydroxy-1,2,4 triazole,
caractérisé en ce que :

a) on a fait réagir des 1-chloro-(thio)-carbonyltriazolinones de formule générale II

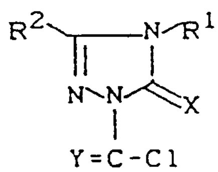 (II)

dans laquelle $R^1$, $R^2$, X et Y ont les significations indiquées ci-dessus,
avec des amines de formule générale III

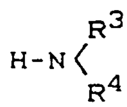 (III)

dans laquelle $R^3$ et $R^4$ ont les significations indiquées ci-dessus,
éventuellement en présence d'un diluant et éventuellement en présence d'un accepteur d'acide ; ou bien

b) lorsque $R^3$ représente l'hydrogène, on fait réagir des triazolinones non substituées en position 1, de formule générale IV

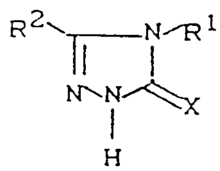 (IV)

dans laquelle $R^1$, $R^2$ et X ont les significations indiquées ci-dessus, avec des iso(thio)cyanates de formule V

$R^4-N=C=Y$ (V)

dans laquelle $R^4$ et Y ont les significations indiquées ci-dessus,
éventuellement en présence d'un diluant et éventuellement en présence d'un produit auxiliaire de réaction.

**3.** Produits herbicides, caractérisés en ce qu'ils contiennent au moins une triazolinone substituée de formule générale I de la revendication 1.

**4.** Procédé pour combattre les végétaux adventices, caractérisé en ce que l'on fait agir sur ces végétaux et/ou leur habitat des triazolinones substituées de formule générale I de la revendication 1.

**5.** Utilisation des triazolinones substituées de formule générale I de la revendication 1, pour la lutte contre les végétaux adventices.

**6.** Procédé de préparation de produits herbicides, caractérisé en ce que l'on mélange des triazolinones de formule générale I de la revendication 1, avec des diluants et/ou des agents tensioactifs.

**7.** Composés de formule générale II

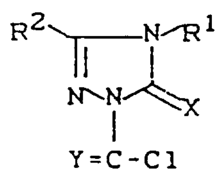

(II)

dans laquelle
$R^1$ représente un groupe méthyle, éthyle, n- ou iso-propyle, n-, iso-, sec- ou tert-butyle, n- ou iso-pentyle, n- ou iso-hexyle, allyle, propargyle, méthoxy, éthoxy, méthoxyméthyle, un groupe halogénoalkyle à chaîne droite ou ramifiée contenant 1 à 4 atomes de carbone et 1 à 9 atomes d'halogènes identiques ou différents, en particulier de fluor, de chlore ou de brome, un groupe cyclopentyle, cyclohexyle, cyclopropyle, cyclopropylméthyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorméthoxy ou trifluorométhylthio,
$R^2$ représente un groupe

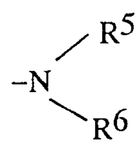

ou un groupe $S(O)_n$-$R^7$
X représente l'oxygène ou le soufre, et
Y représente l'oxygène ou le soufre,
$R^5$ et $R^6$ représentent chacun, indépendamment l'un de l'autre, un groupe méthyle, éthyle, n- ou iso-propyle, n-, iso-, sec- ou tert-butyle, n- ou iso-pentyle, allyle, propargyle, un groupe halogénoalkyle en $C_1$-$C_4$, halogénoalcényle en $C_3$-$C_6$ ou halogénoalcynyle en $C_3$-$C_6$, chacun d'eux à chaîne droite ou ramifiée et contenant chacun 1 à 9 atomes d'halogènes identiques ou différents, un groupe méthoxyméthyle, méthoxyéthyle, méthoxy, éthoxy, cyclopropyle, cyclopropylméthyle, cyclopentyle, cyclohexyle, cyclohexylméthyle, cyclohexyléthyle, cyclopentylméthyle, ou un groupe benzyle, phényle ou phényléthyle, portant chacun le cas échéant 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio, ou bien
$R^5$ et $R^6$ forment ensemble et avec l'atome d'azote auquel ils sont reliées un hétérocycle de formule

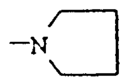 ; 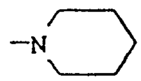 ; 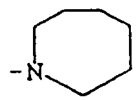 ; 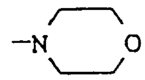 ;

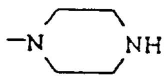 ; 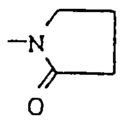 ; 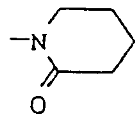 ; 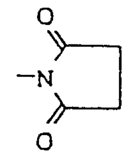 ;

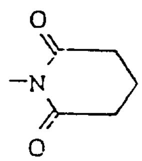 ; 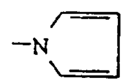 ou 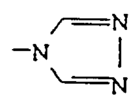

portant éventuellement 1 à 3 substituants identique ou différents choisis parmi les groupes méthyle, éthyle, n- ou iso-propyle, le chlore ou les groupes trifluorométhyle,
$R^7$ représente un groupe méthyle éthyle, n- ou iso-propyle, n-, iso-, sec- ou tert-butyle, allyle, propargyle, cyclopentyle, cyclohexyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy ou trifluorométhyle, et
n est égal à 0,1 ou 2.

**8.** Procédé de préparation des composés de formule générale II

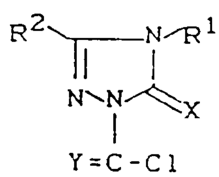 (II)

dans laquelle $R^1$ représente un groupe méthyle, éthyle, n- ou iso-propyle, n-, iso, sec- ou tert-butyle, n- ou iso-pentyle, n- ou iso-hexyle, allyle, propargyle, méthoxy, éthoxy, méthoxyméthyle, un groupe halogénoalkyle à chaîne droite ou ramifiée contenant 1 à 4 atomes de carbone et 1 à 9 atomes d'halogènes identiques ou différents, en particulier de fluor, de chlore ou de brome, un groupe cyclopentyle, cyclohexyle, cyclopropyle, cyclopropylméthyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio,
$R^2$ représente un groupe

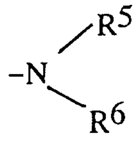

ou un groupe $S(O)_n$-$R^7$
X représente l'oxygène ou le soufre, et
Y représente l'oxygène ou le soufre,

$R^5$ et $R^6$ représentent chacun, indépendamment l'un de l'autre, un groupe méthyle, éthyle, n- ou iso-propyle, n-, iso-, sec- ou tert-butyle, n- ou iso-pentyle, allyle, propargyle, un groupe halogénoalkyle en $C_1$-$C_4$, halogénoalcényle en $C_3$-$C_6$ ou halogénoalcynyle en $C_3$-$C_6$, chacun d'eux à chaîne droite ou ramifiée et contenant chacun 1 à 9 atomes d'halogènes identiques ou différents, un groupe méthoxyméthyle, méthoxyéthyle, méthoxy, éthoxy, cyclopropyle, cyclopropylméthyle, cyclopentyle, cyclohexyle, cyclohexylméthyle, cyclohexyléthyle, cyclopentylméthyle, ou un groupe benzyle, phényle ou phényléthyle, portant chacun le cas échéant 1 à 3 substituants identiques ou différents choisis parmi le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhyle, trifluorométhoxy ou trifluorométhylthio, ou bien
$R^5$ et $R^6$ forment ensemble et avec l'atome d'azote auquel ils sont reliées un hétérocycle de formule

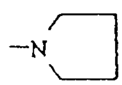 ; 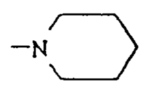 ; 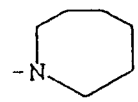 ; 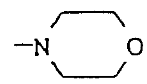 ;

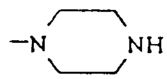 ; 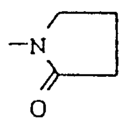 ; 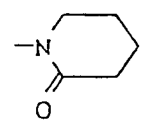 ; 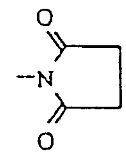 ;

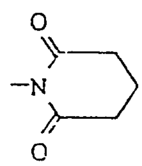 ; 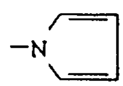 ou 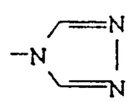

portant éventuellement 1 à 3 substituants identique ou différents choisis parmi les groupes méthyle, éthyle, n- ou iso-propyle, le chlore ou les groupes trifluorométhyle,
$R^7$ représente un groupe méthyle, éthyle, n- ou iso-propyle, n-, iso-, sec- ou tert-butyle, allyle, propargyle, cyclopentyle, cyclohexyle, cyclohexylméthyle, cyclohexyléthyle ou un groupe benzyle ou phényle, chacun d'eux portant éventuellement 1 à 3 substituants identiques ou différents choisis parmi le fluor, le chlore, le brome, les groupes cyano, nitro-méthyle, éthyle, n- ou iso-propyle, n-, iso-, sec- ou tert-butyle, méthoxy, éthoxy ou trifluorométhyle, et
n est égal à 0, 1 ou 2,
caractérisé en ce que :

a) on a fait réagir des triazolinones non substitués en position 1, de formule générale IV

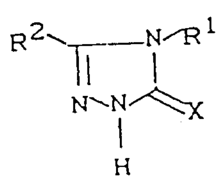 (IV)

dans laquelle $R^1$, $R^2$ et X ont les significations indiquées ci-dessus, avec le (thio)phosgène de formule VI

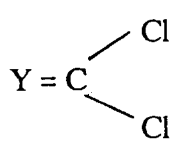 (VI)

dans laquelle Y a les significations indiquées ci-dessus,

éventuellement en présence d'un diluant et éventuellement en présence d'un accepteur d'acide, ou bien

b) on fait réagir des chlorhydrates d'aminoguanidinium de formule générale VII

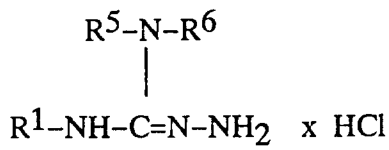

(VII)

dans laquelle $R^1$, $R^5$ et $R^6$ ont les significations indiquées ci-dessus, avec le double de la quantité molaire de (thio)phosphène de formule VI

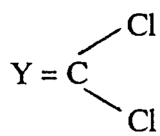

(VI)

dans laquelle Y a les significations indiquées ci-dessus,

éventuellement en présence d'un diluant et éventuellement en présence d'un accepteur d'acide.